# NEUE EUROPÄISCHE PATENTSCHRIFT

(11) **EP 0 528 828 B2**
(45) Veröffentlichungstag und Bekanntmachung des Hinweises auf die Entscheidung über den Einspruch: **03.12.1997**
(45) Hinweis auf die Patenterteilung: 15.06.1994
(21) Anmeldenummer: 91908155.4
(22) Anmeldetag: 08.04.1991
(51) Int. Cl.: C12N 9/20, C12N 15/55, C12N 1/21, C11D 3/386

(54) **ALKALISCHE BACILLUS-LIPASEN, HIERFÜR CODIERENDE DNA-SEQUENZEN SOWIE BACILLI, DIE DIESE LIPASEN PRODUZIEREN**
ALKALINE BACILLUS LIPASES, CODING DNA SEQUENCES THEREFOR AND BACILLI WHICH PRODUCE THESE LIPASES
LIPASES BACILLAIRES ALCALINES, SEQUENCES D'ADN DE CODAGE POUR CELLES-CI ET BACILLES PRODUISANT CES LIPASES

(30) Priorität: 14.04.1990 DE 4012070
(43) Veröffentlichungstag der Anmeldung: 03.03.1993
(73) Patentinhaber: Genencor International GmbH, 31582 Nienburg/Weser (DE)
(72) Erfinder: MÖLLER, Bernhard, D-3000 Hannover (DE); VETTER, Roman, D-3167 Burgdorf (DE); WILKE, Detlef, D-3015 Wennigsen (DE); FOULLOIS, Birgit, D-3000 Hannover 91 (DE)
(74) Vertreter: Brandes, Jürgen, Dr.
(86) Internationale Anmeldenummer: EP9100664
(87) Internationale Veröffentlichungsnummer: WO9116422

(56) Entgegenhaltungen:
- EP-A- 0 334 462
- Chemical Abstracts, vol. 94, no. 23, 8 June 1981, (Columbus, Ohio, US), A. Mourey: "Lipolytic activity of Bacillus pumilus", p. 301, abstract 188319d
- Chemical Abstracts, vol. 113, no. 15, 8 October 1990, (Columbus, Ohio, US), p. 521, abstract 130743g
- Agr. Biol. Chem. vol. 39, 1975 p. 1063-1070
- J. Biochem., vol. 107, 1990 p. 426-430

## Beschreibung

Die vorliegende Erfindung bezieht sich auf alkalische Bacillus-tipasen und hierfür codierende DNA-Sequenzen, auf die Verwendung und ein Verfahren zur Herstellung dieser Lipasen, sowie auf Bacillus-Stämme, die die Fähigkeit besitzen, diese Lipasen zu bilden.

Enzymatische Zusammensetzungen für Wasch-, Reinigungs- und Bleichanwendungen sind im Stand der Technik gut bekannt Zwar wurden für diese Anwendungen bereits vielfältige Arten von Enzymen vorgeschlagen, doch richtete sich das Hauptinteresse hierbei im wesentlichen auf Proteasen und Amylasen. Die im Stand der Technik für Wasch-, Reinigungs- und Bleichzusammensetzungen bisher vorgeschlagenen Lipasen wurden durch Kultivierung von Mikroorganismen wie Pseudomonas-, Rhizopus-, Chromobacter und Humicola-Arten (inklusive Thermomyces-Spezies) erhalten.

Obwohl solche Lipasen als mögliche Enzyme für die genannten Anwendungen in Erwägung gezogen wurden, haben sich Lipasen in Wasch-, Reinigungs- und Bleichzusammensetzungen bisher kaum durchgesetzt, da sich verschiedene Inhaltsstoffe dieser Zusammensetzungen negativ auf die Aktivität der Lipasen auswirken. Beispielsweise ist es bekannt, daß insbesondere anionische synthetische Tenside die Lipaseaktivität negativ beeinflussen. Von der Vielzahl der Enzyme des Standes der Technik, die zur Klasse der Lipasen gehören, hat zwar jedes einzelne Enzym auch spezielle vorteilhafte Eigenschaften, dennoch sind die Anwendungsmöglichkeiten für diese Enzyme aufgrund ihrer nachteiligen Eigenschaften nur begrenzt

Ferner unterliegen die Lipasen, da sie selbst auch Proteine sind, dem proteolytischen Abbau durch Waschmitteiproteasen, sofern sie in Kombination mit diesen Proteasen als waschaktive Bestandteile von Wasch-, Reinigungs- und Bleichmittelzusammensetzungen eingesetzt werden. Hierdurch besteht die Gefahr, daß bei gemeinsamer Verwendung von Protease und Lipase in diesen Zusammensetzungen die lipolytische Aktivität aufgrund von Aktivitätsverlusten ganz oder teilweise nicht genutzt werden kann.

Andererseits werden aber gerade bei niedrigen Wasch- und Reinigungstemperaturen, beispielsweise bei Temperaturen bis 40 °C, Fette und Öle durch Wasch- und Reinigungszusammensetzungen des Standes der Technik nur schlecht und unvollständig von den zu reinigenden Geweben und/oder Gegenständen entfernt, sofern der Wasch- und Reinigungsvorgang nicht durch lipolytisch wirksame Enzyme unterstützt wird.

Es bestand daher die Aufgabe, solche Lipasen bereitzustellen, die als Additiv für Waschmittel-, Reinigungsmittel- und Bleichmittelzusammensetzungen nützlich sind, die eine hohe Aktivität bei Temperaturen bis 40 °C besitzen und vielseitige Verwendbarkeit in Gegenwart von Proteasen und in einem weiten, insbesondere neutralen bis alkalischen, pH-Bereich gestatten.

Überraschenderweise wurde nunmehr gefunden, daß Lipasen, die von Bacillus-Arten ausgeschieden werden, mit einem pH-Optimum im alkalischen pH-Bereich und einem Temperatur-Optimum im Bereich von etwa 30 bis 40 °C, die gewünschten Eigenschaften aufweisen. In zweckmäßigen Ausgestaltungen der Erfindung handelt es sich insbesondere um alkalische Bacillus-Lipasen, die durch Kultivierung von Bacillus pumilus erhältlich sind.

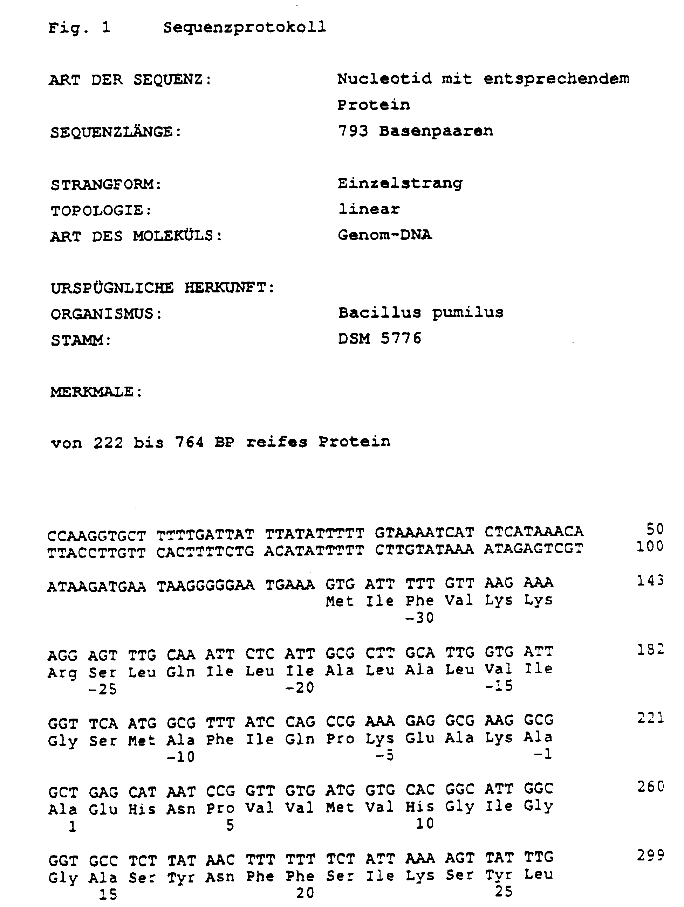

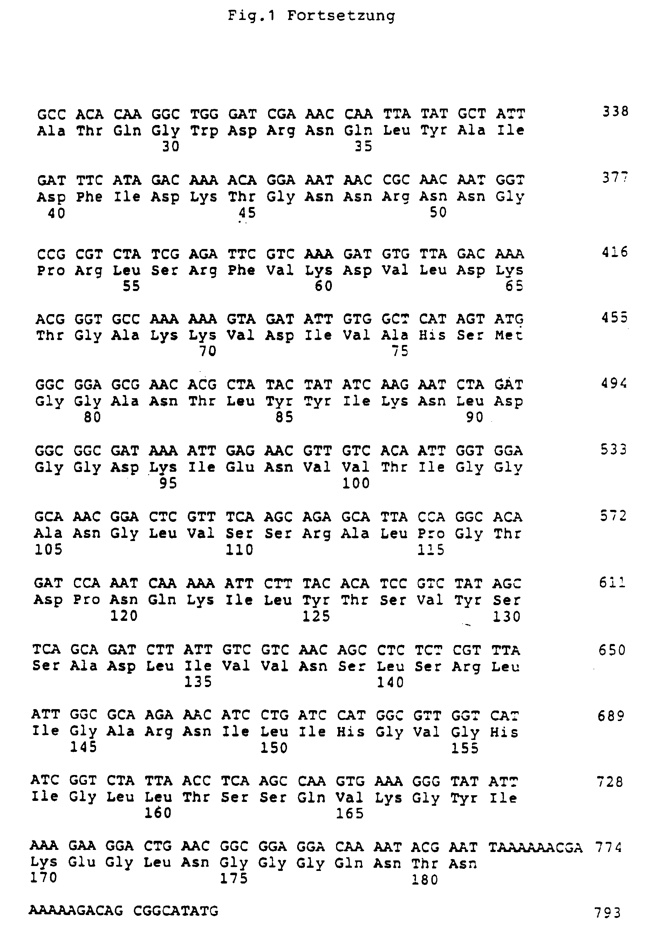

In bevorzugten Ausgestaltungen der Erfindung liegen insbesondere solche alkalischen Bacillus-Lipasen der vorstehend angegebenen Art vor, die eine Aminosäurensequenz besitzen, die wenigstens 70 %, vorzugsweise wenigstens 80 %, insbesondere aber wenigstens 90 %, Homologie zu der in Fig. 1 angegebenen Aminosäurensequenz aufweist. Unter Homologie wird hier der Grad der Verwandtschaft der betreffenden Aminosäurensequenz einer Bacillus-Lipase zu den Aminosäurensequenzen der Lipasen aus den Bacillus-Naturisolaten DSM 5776, DSM 5777 oder DSM 5778, insbesondere zu der Aminosäurensequenz der Lipase aus dem Bacillus-Naturisolat DSM 5776 wie sie in Fig. 1 angegeben ist, verstanden. Zur Bestimmung der Homologie werden jeweils die einander entsprechenden Abschnitte der Aminosäurensequenz der Lipasen aus den Bacillus-Naturisolaten, insbesondere die Abschnitte der Aminosäurensequenz der Fig. 1, und einer damit zu vergleichenden Aminosäurensequenz einer Bacillus-Lipase so zur Deckung miteinander gebracht, daß maximale Übereinstimmung zwischen den Aminosäurensequenzen besteht, wobei durch Deletion oder Insertion einzelnerAminosäuren verursachte Unterschiede berücksichtigt und durch entsprechende Verschiebungen von Sequenzabschnitten ausgeglichen werden. Die Zahl der nunmehr in den Sequenzen miteinander übereinstimmenden Aminosäuren ("homologe Positionen"), bezogen auf die Gesamtzahl der in der Sequenz einer der Lipasen aus den genannten Bacillus-Naturisolaten enthaltenen Aminosäuren, ergibt dabei die Homologie in %. Abweichungen in den Sequenzen können sowohl durch Variation, Insertion als auch Deletion von Aminosäuren bedingt sein.

In einer sehr bevorzugten Ausgestaltung der Erfindung sind die erfindungsgemäßen Lipasen insbesondere alkalische Bacillus-Lipasen, die durch Kultivierung von Bacillus pumilus der Spezies DSM 5776, DSM 5777 oder DSM 5778 erhältlich sind und beispielsweise wie weiter unten beschrieben isoliert werden können.

Die durch die Erfindung vorgeschlagenen neuen alkalischen Bacillus-Lipasen weisen insbesondere die folgenden weiteren Eigenschaften auf:
(1) Wirkung: Abbau von Triacylglyceriden und von Fettsäureestern;
(2) pH-Optimum: etwa bei pH-Werten von 9 bis 10;
(3) pH-Stabilität: bei pH-Werten von 6,5 bis 11 erweisen sich die Enzyme als völlig stabil; nach Inkubation bei pH 11 und 4 °C für 21 h beträgt die Restaktivität der Enzyme wenigstens 90 %;
(4) Temperatur-Optimum: etwa 30 bis 40 °C;
(5) Temperatur-Stabilität: durch Inkubation der Enzyme bei Temperaturen bis 40 °C für 30 Minuten werden die Enzyme nicht wesentlich in ihrer Aktivität beeinträchtigt; nach 30 Minuten Inkubation bei 40 °C beträgt die Restaktivität der Enzyme wenigstens 90 %.

Die erfindungsgemäßen Bacillus-Lipasen eignen sich vorteilhaft als Additive für Waschmittel- und Reinigungsmittelzusammensetzungen etc., die neutrale bis alkalische pH-Werte besitzen und bei niedrigen Temperaturen, insbesondere bei Temperaturen bis 40 °C angewendet werden sollen. Die Erfindung betrifft daher auch die Verwendung der erfindungsgemäßen alkalischen Bacillus-Lipasen in Waschmittel-, Reinigungsmittel-, Bleichmittel- oder Geschirrspülmittelzusammensetzungen. Sie können hierbei auch vorteilhaft in Gegenwart von anderen üblichen Enzymen, insbesondere auch in Gegenwart von Proteasen, eingesetzt werden. Eine ganz bevorzugte Verwendung der erfindungsgemäßen alkalischen Bacillus-Lipasen betrifft deren Verwendung in Waschmittel-, Reinigungsmittel-, Bleichmittel- oder Geschirrspülmittelzusammensetzungen für niedrige Anwendungstemperaturen, vorzugsweise für Anwendungstemperaturen von etwa 30 bis 40 °C. Für diese Anwendungen stellt die Erfindung eine Gruppe neuer alkalischer Lipasen aus Bacillus-Arten mit verbesserten Eigenschaften zur Verfügung, deren Verwendung auch zu vorteilhaften Waschmittel-, Reinigungsmittel-, Geschirrspülmittel- und Bleichmittelzusammensetzungen führt. Die Erfindung umfaßt daher weiterhin diese vorteilhaften Zusammensetzungen zum Waschen, Reinigen, Bleichen oder Geschirrspülen, die eine der erfindungsgemäßen alkalischen Bacillus-Lipasen enthalten. Ausgestaltungen dieser Zusammensetzungen enthalten die erfindungsgemäßen alkalischen Bacillus-Lipasen in Gegenwart von Protease. Bevorzugte Ausgestaltungen der vorstehenden Zusammensetzungen zeichnen sich dadurch aus, daß sie die alkalischen Bacillus-Lipasen in einer Formulierung für niedrige Anwendungstemperaturen, vorzugsweise für Anwendungstemperaturen von etwa 30 bis 40 °C, enthalten.

Die erfindungsgemäßen Lipasen können in Wasch- und Reinigungsmittelformulierungen, beispielsweise in Pulverwaschmittelformulierungen, einzeln oder gewünschtenfalls auch in Kombination miteinander, gegebenenfalls auch in Kombination mit Wasch- und Reinigungsmittelproteasen des Standes der Technik oder anderen in solchen Zusammensetzungen üblichen Enzymen, wie z.B. Amylasen, Lipasen, Pektinasen, Nukleasen, Oxidoreduktasen etc., eingesetzt werden. Die erfindungsgemäßen Lipasen werden in den Wasch- und Reinigungsmittelformulierungen in an sich für Waschmittelenzyme üblichen Mengen (geeignet sind bspw. bereits Mengen ab etwa 0,1 Gew.-%), insbesondere in einer Menge bis zu 3 Gew. -% (bezogen auf die Trockensubstanz der Gesamtzusammensetzung), vorzugsweise in einer Menge von 0,2 bis 1,5 Gew. -%, verwendet.

Außer den bereits erwähnten Waschmittelenzymen können die Wasch- und Reinigungsmittel der Erfindung alle an sich im Stand der Technik üblichen Waschmittelinhaltsstoffe wie Tenside, Bleichmittel oder Gerüststoffe (Builder), sowie weitere übliche Hilfsstoffe für die Formulierung von Waschmitteln in an sich üblichen Mengen enthalten. Zu den Hilfsstoffen gehören z.B. Verstärker, Enzymstabilisatoren, Schmutzträger und/oder Kompatibilisierungsmittel, Komplex- und Chelatbildner, Seifenschaumregulatoren und Zusatzstoffe wie optische Aufheller, Opazifizierungsmittel, Korrosionsinhibitoren, Antielektrostatika, Farbstoffe, Bakterizide, Bleichmittelaktivatoren, Persäurebleichmittelvorstufen.

So enthalten erfindungsgemäße Waschmittelformulierungen in typischer beispielhafter Zusammensetzung bezogen auf Trockensubstanz
a) wenigstens 5 Gew.-%, z.B. 10 bis 50 Gew.-%, eines Tensids oder Tensidgemisches
b) bis zu 40 Gew. -% eines Builders oder eines Builder-Gemisches,
c) bis zu 40 Gew.-% eines Bleichmittels oder Bleichmittelgemisches, vorzugsweise ein Perborat wie Natriumperborat-Tetrahydrat oder Natriumperborat-Monohydrat,
d) 0,1 bis-3 Gew.-%, vorzugsweise 0,2 bis 1,5 Gew.-% wenigstens einer erfindungsgemäßen Lipase und ggf. 0,1 bis 3 Gew.-% einer Protease
e) weitere Bestandteile wie Hilfsstoffe etc. ad 100 Gew.-%

Solche Waschmittelformulierungen können in an sich üblicher Weise formuliert werden. Die erfindungsgemäßen Lipasen können dazu z.B. in Form von Granulaten, Prills oder Pellets, gegebenenfalls auch mit Oberflächenüberzügen versehen, mit den anderen Komponenten der Waschmittelformulierung in an sich bekannter Weise vermischt werden.

Die erfindungsgemäßen Bacillus-Lipasen eignen sich darüber hinaus sehr gut für den Einsatz in an sich üblichen flüssigen Reinigungsformulierungen, z.B. in Geschirrspülmitteln oder in Flüssigwaschmittelformulierungen. In diese Formulierungen können die erfindungsgemäßen Lipasen auch in Form von Flüssigenzymformulierungen eingebracht werden.

Die alkalischen Bacillus-Lipasen der Erfindung können erhalten werden, indem man ein Bakterium, welches zum Genus Bacillus gehört und welches zur Bildung der alkalischen Lipase fähig ist, in an sich üblicher Weise zunächst kultiviert und man danach die Zellen z.B. durch Filtration oder durch Zentrifugation abtrennt, das Enzym durch Membranfiltration oder Fällung konzentriert, aufreinigt, ggf. auch isoliert und einer gewünschten Verwendung zuführt.

Für die Herstellung und Gewinnung der erfindungsgemäßen Lipasen erweisen sich z.B. einerseits die Naturisolate von Bacillus-Stämmen, die die erfindungsgemäßen Lipasen produzieren, selbst als geeignet. Solche Bacilli lassen sich aus der Natur isolieren, indem man beispielsweise tierisches oder pflanzliches Fett enthaltendes Material zunächst einige Zeit unter für Lipase ausscheidende Bacillus-Spezies günstigen Bedingungen lagert, danach gegebenenfalls pasteurisiert und anschließend mit der so erhaltenen Probe ein für das Wachstum von Lipase ausscheidenden Bacillus-Spezies geeignetes Medium beimpft. Nach einer ausreichenden Inkubationszeit werden vegetative Zellen durch Hitzebehandlung der Kultur abgetötet und die so behandelte Kultur, gegebenenfalls nach Einstellung einer geeigneten Verdünnung, beispielsweise auf Lipase-Screeningplatten ausgespatelt und die Platten nachfolgend inkubiert. Nach einer ausreichenden Inkubationsdauer werden die Lipase-Screeningplatten dann in an sich bekannter Weise auf Lipase-bildende Kolonien untersucht und diese Kolonien in an sich bekannter Weise isoliert. In dieser Weise können geeignete, eine alkalische Bacillus-Lipase bildende Bacillus-Naturisolate erhalten werden. Beispiele sind Spezies von Bacillus pumilus, insbesondere die ebenfalls einen Erfindungsgegenstand darstellenden Bacillus-Naturisolate, die am 07.02.1990 unter den Nummern DSM 5776, DSM 5777 und DSM 5778 bei der Deutschen Sammlung von Mikroorganismen, Bundesrepublik Deutschland, hinterlegt wurden.

Andererseits können in vorteilhafter Weise, insbesondere aus Gründen der Produktionsvereinfachung und -optimierung sowie zur Ausbeutesteigerung, auch andere Bacillus-Stämme, in die zuvor durch Transformation die notwendige genetische Information über die erfindungsgemäßen Lipasen und deren Expression eingebracht wurde, zur Herstellung und Gewinnung der erfindungsgemäßen Bacillus-Lipasen eingesetzt werden.

Die Erfindung umfaßt daher auch ein Verfahren zur Herstellung der erfindungsgemäßen alkalischen Lipasen mit transformierten Mikroorganismen, vorzugsweise mit transformierten Bacilli, welche einen Vektor mit einer solchen DNA-Sequenz enthalten, welche für eine Aminosäurensequenz einer der weiter oben beschriebenen erfindungsgemäßen, alkalischen Bacillus-Lipasen codiert. Der erfindungsgemäß transformierte Mikroorganismus wird wie oben angegeben kultiviert und aus dem Kulturmedium die alkalische Bacillus-Lipase isoliert. Bevorzugte transformierte Mikroorganismen für die Herstellung und Gewinnung der erfindungsgemäßen Bacillus-Lipasen sind Bacillus-Spezies wie Bacillus subtilis, Bacillus alcalophilus, Bacillus licheniformis oder Bacillus amyloliquefaciens. Die zur Expression von Bacillus-Lipasen geeigneten, erfindungsgemäß transformierten Mikroorganismen zeichnen sich dadurch aus, daß sie mit einem Vektor transformiert sind, der die genetische Information für eine der erfindungsgemäßen alkalischen Bacillus-Lipasen enthält Die genetische Information für diese erfindungsgemäßen Bacillus-Lipasen wird hierbei jeweils durch eine DNA-Sequenz gegeben, die für eine alkalische Bacillus-Lipase mit einer Aminosäuren-Sequenz codiert, die wenigstens 70 %, vorzugsweise wenigstens 80 %, insbesondere aber wenigstens 90 %, Homologie zu der in Fig. 1 angegebenen Aminosäurensequenz aufweist. Diese neuen DNA-Sequenzen, die zur Transformation von Mikroorganismen geeigneten Vektoren, vorzugsweise Expressionsvektoren, die diese neuen DNA-Sequenzen enthalten sowie die mit diesen Vektoren transformierten Mikroorganismen, vorzugsweise derart transformierte Bacilli wie insbesondere der oben angegebenen Art, sind ebenfalls ein Gegenstand der Erfindung.

Zur Erzeugung der im vorstehenden Verfahren eingesetzten, transformierten Mikroorganismen kann so vorgegangen werden, daß man
a) zunächst aus einem geeigneten Bacillus-Stamm, der eine alkalische Lipase mit einer Aminosäurensequenz mit wenigstens 70 %, vorzugsweise wenigstens 80 %, insbesondere aber wenigstens 90 %, Homologie zu der in Fig. 1 angegebenen Aminosäurensequenz produziert, die für die Lipase codierende DNA-Sequenz (d.h. das Strukturgen der Lipase) isoliert,
b) ggf. die Nukleotidabfolge dieser DNA-Sequenz zur weiteren Identifizierung der Lipase bestimmt,
c) nachfolgend mit Hilfe der isolierten DNA-Sequenz einen Expressionsvektor herstellt und
d) den erhaltenen Expressionsvektor in einen geeigneten Mikroorganismus, welcher schließlich zur Produktion der alkalischen Lipase eingesetzt werden kann, transformiert.

Die Verfahrensschritte zur Isolierung und Gewinnung der erfindungsgemäßen alkalischen Lipasen nach dem vorstehenden Verfahren, sowie die hierbei erhaltenen, zum Teil ebenfalls einen Erfindungsgegenstand darstellenden Zwischenprodukte in Form von DNA-Sequenzen bzw. DNA-lnserts mit dem Lipase-Gen, Vektoren, insbesondere Expressionsvektoren, und transformierten Mikroorganismen werden nachfolgend im einzelnen näher beschrieben.

Die Strukturgene, die fürAminosäurensequenzen der alkalischen Bacillus-Lipasen codieren, können nach an sich bekannten, allgemeinen Methoden erhalten werden. Hierzu wird z.B. aus einem Bacillus ("Donor-Bacillus"), der eine alkalische Lipase produziert, insbesondere aus einem Bacillus aus der Gruppe DSM 5776, DSM 5777 oder DSM 5778, die chromosomale DNA nach an sich bekannten Methoden isoliert und mit geeigneten Restriktionsendonukleasen partiell hydrolysiert. Restriktionsendonukleasen sind Enzyme, die substratsspezifisch doppelsträngige DNA dadurch in Fragmente zerlegen, daß sie Phosphodiesterbindungen zwischen einzelnen Nukleotidbausteinen der DNA spalten. Alle Restri ktionsendonukleasen vermögen bestimmte Basensequenzen der DNA zu erkennen, welche für die Aktivität der betreffenden Restriktionsendonukleasen spezifische Wirkungsorte (Schnittstellen) markieren. Beim Schneiden (Restriktion) doppelsträngiger DNA, wie hier, entstehen bei einigen Restriktionsendonukleasen spezifische, sogenannte "überstehende Enden", die unter bestimmten Renaturierungsbedingungen wieder miteinander oder mit entsprechenden (komplementären) überstehenden Enden anderweitig gewonnener DNA-Fragmente verbunden (ligiert) werden können (Rekombination). Beim Schneiden mit anderen Restriktionsendonukleasen entstehen DNA-Doppelstränge mit glatten Enden. Diese DNA-Doppelstränge mit glatten Enden können mit beliebigen DNA-Doppelsträngen, die ebenfalls glatte Enden besitzen, rekombiniert werden.

Die erhaltenen Restriktionsfragmente der Donor-DNA können durch Gelelektrophorese oder Zentrifugation durch einen Saccharase-Dichte-Gradienten nach Größe aufgetrennt und die Fragmente gewünschter Größe dann mit einer geeigneten, doppelsträngigen Vektor-DNA rekombiniert werden. Vektoren sind DNA-Moleküle, die sich als Transportmoleküle (Vehikel) zur Einschleusung (Transformation) von Fremd-DNA in Wirtszellen eignen, dort ggf. autonom replizierbar sind und gegebenenfalls noch sogenannte Marker besitzen. Marker sind DNA-Fragmente, die für bestimmte, beobachtbare Eigenschaften (z.B. Antibiotika-Resistenz) codieren und der nachfolgenden Selektion der transformierten Mikroorganismen (Transformanten) dienen. Häufig verwendete Vektoren sind die sogenannten Plasmide, d.h. extrachromosomale, ringförmige, doppelsträngige Bakterien-DNA, die sich durch geeignete Methoden in andere Mikroorganismen einbringen läßt und dort vermehrbar ist. Ein hierverwendetes Plasmid mit der Bezeichnung pUB110 kann, wie in den Beispielen näher beschrieben, aus dem kommerziell erhältlichen Bacillus subtilis BD366 isoliert werden.

Die vorstehend erhaltene, in vitro rekombinierte DNA kann nun in geeignete Wirtszellen, z.B. in den hier verwendeten, kommerziell erhältlichen Stamm Bacillus subtilis PSL 1 eingebracht werden. Transformanten können mit Hilfe von bekannten Markern auf der Vektor-DNA (z.B. Neomycin-Resistenz) selektiert werden. Unter diesen antibiotikaresistenten Transformanten kann nach Lipase-ausscheidenden Klonen, d.h. genetisch identischen Transformanten, gesucht werden. Aus einem Klon mit Lipaseaktivität wird schließlich die in diese Transformante eingeführte Plasmid-DNA isoliert und durch erneute Transformation eines Bakteriums, insbesondere einer Bacillus-Spezies, überprüft, ob die Lipaseaktivität Plasmid-gebunden und mit der Markereigenschaft gekoppelt ist.

Das so isolierte Plasmid enthält neben der Vektor-DNA (hier insbesondere aus dem Plasmid pUB110) mit bekannten Restriktionsstellen das gewünschte Strukturgen für die gesuchte alkalische Bacillus-Lipase und gegebenenfalls weitere, hier aber nicht benötigte DNA-Sequenzen aus dem Donor-Bacillus. Beispiele für diese Vektoren mit Lipasegen-haltigen Fragmenten (Inserts) sind die Plasmide mit den Bezeichnungen pL2-22-11, pL4-23-14 und pL11-8-20. Die für die jeweiligen Bacillus-Lipasen codierenden DNA-Sequenzen und die zugehörigen Aminosäurensequenzen in diesen Plasmiden können mit Hilfe an sich bekannter Methoden des Standes der Technik sequenziert werden. Man erhält so z.B. die in Fig. 1 angegebene DNA-Sequenz bzw. die zugehörige Aminosäurensequenz.

Die Fähigkeit dieser Plasmide, beispielsweise der Plasmide pL2-22-11, pL4-23-14 und pL11-8-20 zur Expression der alkalischen Bacillus-Lipase kann überprüft werden, indem man ein Bakterium, insbesondere eine Bacillus-Spezies mit einem dieser Plasmide transformiert und die so erhaltenen Transformanten kultiviert und auf Lipaseaktivität überprüft Die erhaltenen Transformanten können darüber hinaus auch zur Herstellung und Gewinnung der erfindungsgemäßen alkalischen Bacillus-Lipasen kultiviert werden, wobei dann die weiter oben beschriebenen erfindungsgemäßen alkalischen Bacillus-Lipasen erhalten werden.

Die erfindungsgemäßen Bacillus-Lipasen zeichnen sich durch vorteilhafte Eigenschaften aus. Sie besitzen eine günstige pH-Stabilität in einem weiten Bereich von pH 5 bis 11 und sind insbesondere im pH-Bereich von 6,5 bis 11 völlig stabil. Das pH-Optimum der erfindungsgemäßen Bacillus-Lipasen liegt in einem für die Anwendung in Wasch- und Reinigungsmittelzusammensetzungen günstigen Bereich von pH 9 bis 10. Ferner besitzen die erfindungsgemäßen Lipasen ein Temperatur-Optimum im Bereich von 30 bis 40 °C. Ferner zeigen sie hervorragende Stabilitäten auch in Waschlauge, sowie auch in Gegenwart von Waschmittelproteasen. Aufgrund ihrer günstigen Aktivität bei Temperaturen bis zu 40 °C eignen sich die erfindungsgemäßen Bacillus-Lipasen insbesondere für die Anwendung in Reinigungs- und Waschmittelzusammensetzungen, die bei niedrigen Temperaturen, insbesondere bis 40 °C angewendet werden sollen. Solche Wasch- und Reinigungsmittelzusammensetzungen, die eine erfindungsgemäße Lipase enthalten, zeigen eine ausgezeichnete Waschwirksamkeit hinsichtlich zu entfernender Öle und/oder Fette.

### Erläuterungen zu den Figuren:

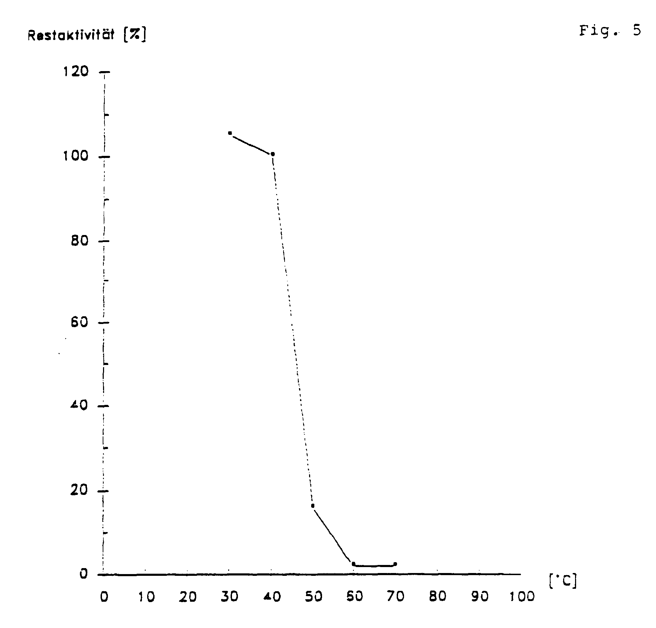

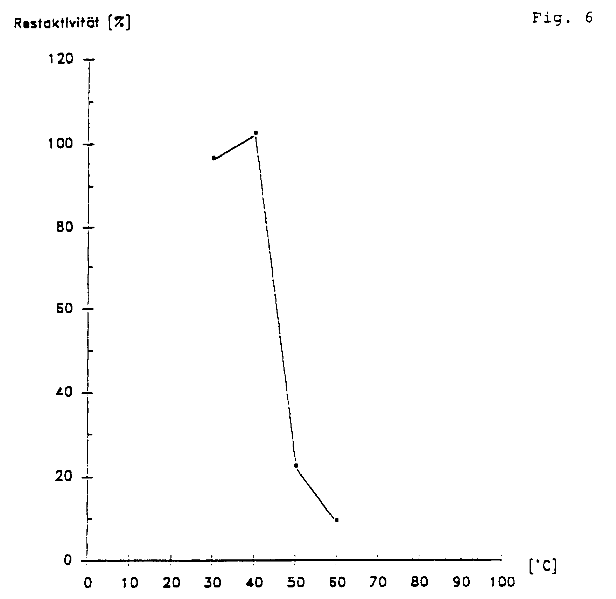

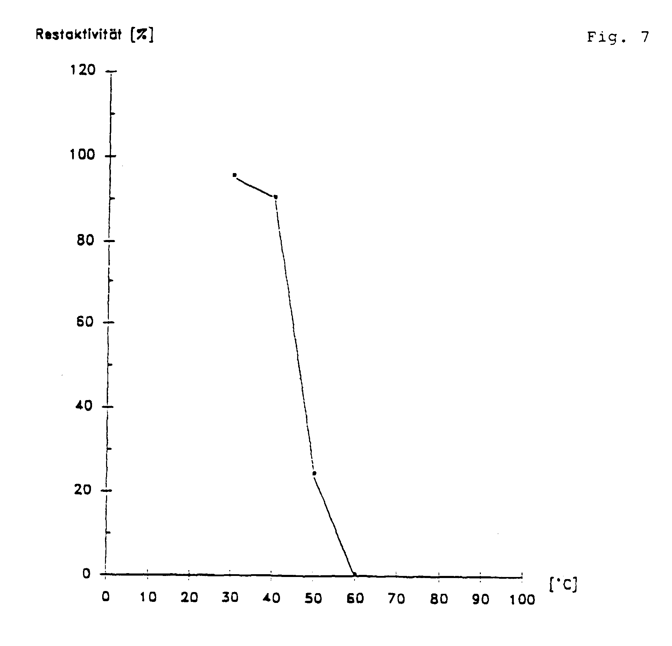

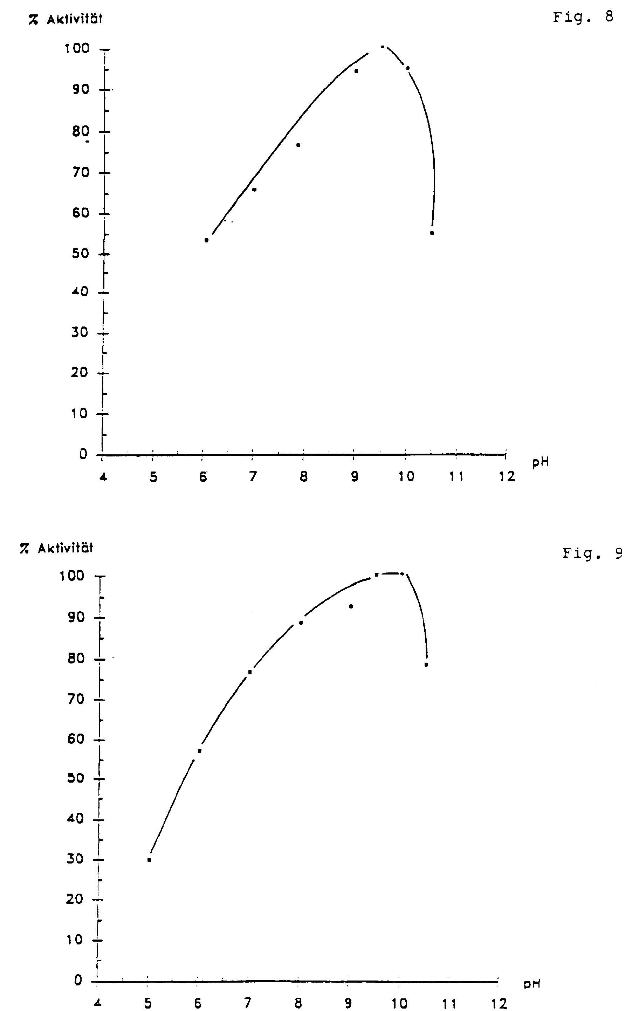

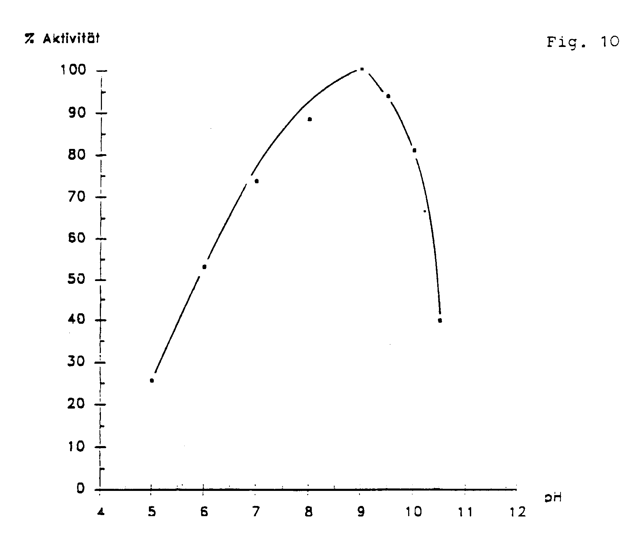

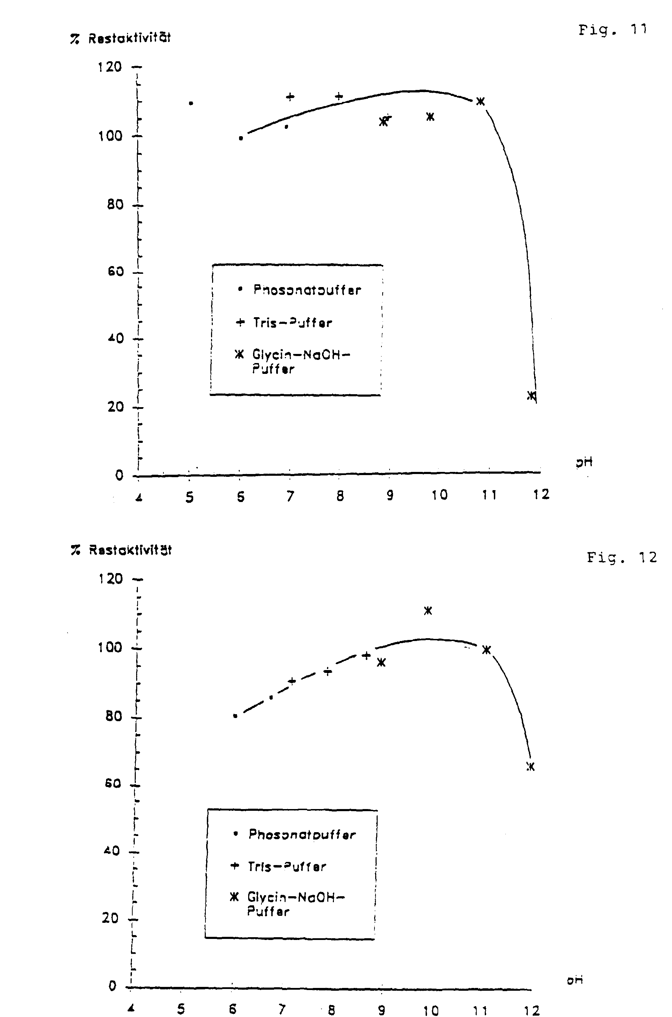

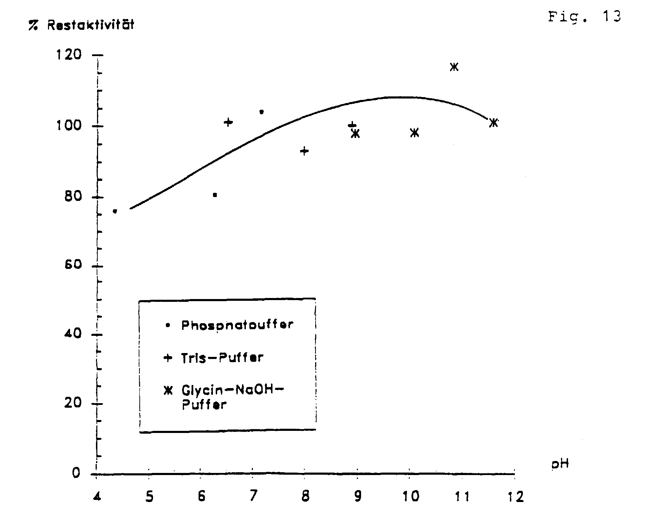

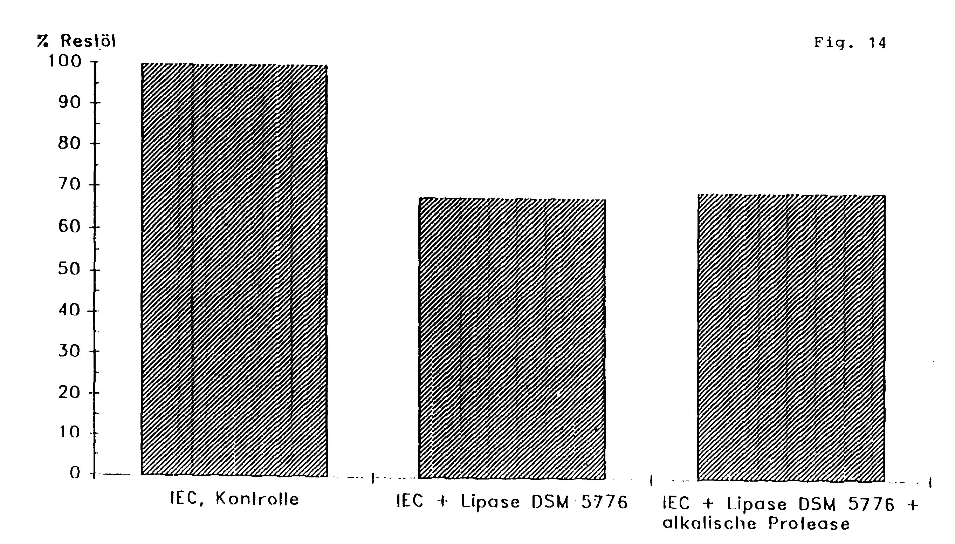

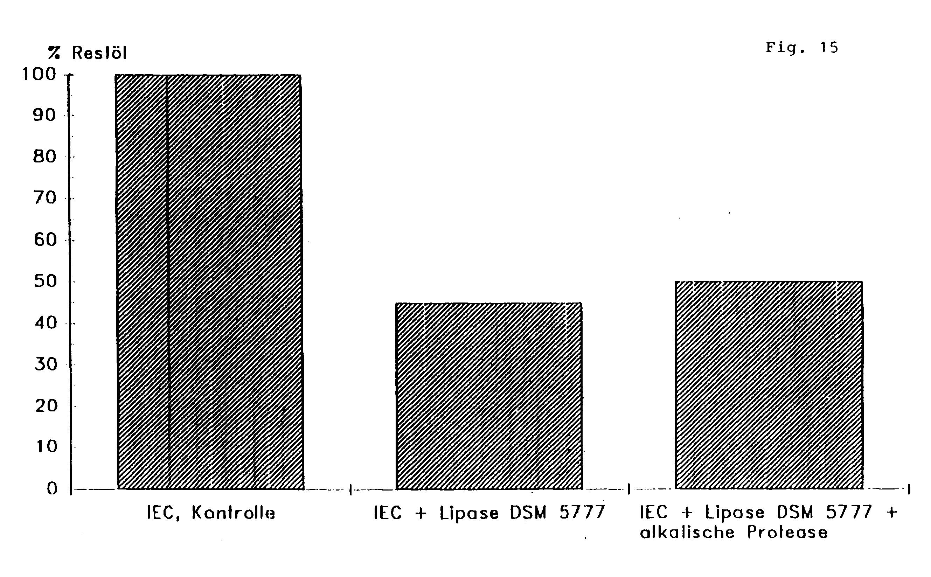

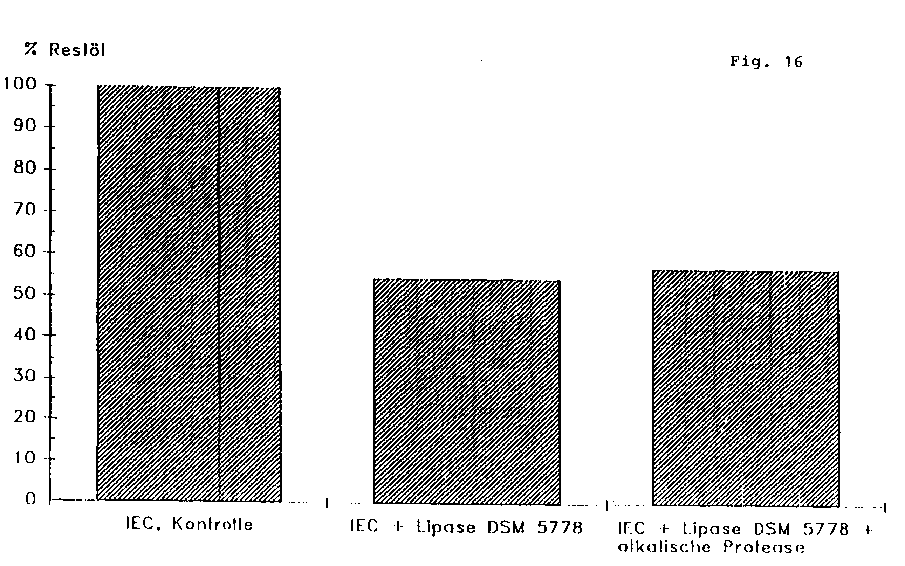

Figur 1:
   DNA-Sequenzprotokoll und zugehörige Aminosäurensequenz der Lipase aus Bacillus pumilus DSM 5776
Figur 2 bis Figur 4:
   Kurven für die Temperatur-Optima der Lipasen aus Bacillus pumilus DSM 5776 (Fig. 2), aus Bacillus pumilus DSM 5777 (Fig. 3) und aus Bacillus pumilus DSM 5778 (Fig. 4).
Figur 5 bis Figur 7:
   Kurven für die Temperatur-Stabilität der Lipasen aus Bacillus pumilus DSM 5776 (Fig. 5), aus Bacillus pumilus DSM 5777 (Fig. 6) und aus Bacillus pumilus DSM 5778 (Fig. 7).
Figur 8 bis Figur 10:
   Kurven für die pH-Optima der Lipasen aus Bacillus pumilus DSM 5776 (Fig. 8), aus Bacillus pumilus DSM 5777 (Fig. 9) und aus Bacillus pumilus DSM 5778 (Fig. 10).
Figur 11 bis Figur 13:
   Kurven für die pH-Stabilität der Lipasen aus Bacillus DSM 5776 (Fig. 11), aus Bacillus DSM 5777 (Fig. 12) und aus Bacillus DSM 5778 (Fig. 13). Zur pH-Wert-Einstellung wurden benutzt Posphatpuffer (·), Tris-HCl-Puffer (+) bzw. Glycin-NaOH-Puffer (*).
Figur 14 bis Figur 16:
   Graphische Darstellung der Waschwirksamkeit von Bacillus-Lipasen in einer IEC-Standardwaschmittelformulierung, sowie in Gegenwart von alkalischer Protease; Waschwirksamkeit der Lipase aus Bacillus pumilus DSM 5776 (Fig. 14), aus Bacillus pumilus DSM 5777 (Fig. 15) und aus Bacillus pumilus DSM 5778 (Fig. 16).

### Beispiele

Die nachfolgende Offenbarung gibt zur weiteren Erläuterung der Erfindung typische beispielhafte Ausgestaltungen der Erfindung wieder, ohne jedoch die Erfindung in ihrem Umfange zu beschränken.

Um die Beispiele zu vereinfachen werden einige häufig wiederkehrende Methoden und Begriffe im folgenden näher erläutert und dann in den einzelnen Beispielen nur noch durch eine Kurzbezeichnung referiert. Sofern nicht anders angegeben, wurde generell nach Methoden gearbeitet, wie sie in Maniatis et al. (Maniatis et al. = T. Maniatis, E. F. Fritsch, J. Sambrook, Molecular Cloning, A Laboratory Manual, Cold Spring Harbor Laboratory, 1982) beschrieben sind.

Die verschiedenen benutzten Restriktionsendonukleasen gehören zum Stand der Technik und sind kommerziell verfügbar. Die bei Verwendung dieser bekannten Restriktionsendonukleasen jeweils erforderlichen Reaktions-, Kofaktor- und übrigen Bedinungen sind ebenfalls bekannt. Z.B. kann für eine Menge von etwa 1 µg DNA eine Einheit (= 1 U = unit) der Restriktionsendonuklease in etwa 20 µl einer Pufferlösung eingesetzt werden. Ausreichende Inkubationszeiten von etwa einer Stunde bei 37 °C wurden gewöhnlich eingehalten, die Inkubationsbedingungen können aber den gegebenen Erfordernissen angepaßt werden. Nach Inkubation mit einer Restriktionsendonuklease wurde das Protein durch Extraktion (z.B. mit Phenol und Chloroform) entfernt und die geschnittene DNA (z.B. aus der wäßrigen Fraktion durch Fällung mit Ethanol) isoliert und der weiteren Verwendung zugeführt

An das Schneiden von DNA oder Vektoren mit Restriktionsendonukleasen kann sich gegebenenfalls eine Hydrolyse des terminalen 5'-Phosphatrestes mit einer alkalischen Phosphatase (Dephosphorylierung) anschließen. Dadurch kann verhindert werden, daß die beim Schneiden entstandenen Enden des restringierten Vektors mit sich selbst rekombinieren und somit die gewünschte Insertion eines Fremd-DNA-Fragmentes in die Restriktionsstelle verhindert würde. Sofern in den Beispielen eine Dephosphorylierung des 5'-Endes vorgenommen wurde, geschah dieses in an sich bekannter Weise. Weitere Angaben zur Durchführung einer DePhosphorylierung und zu dafür benötigten Reagentien können Maniatis et al. (S. 133 - 134) entnommen werden.

Partielle Hydrolyse bedeutet unvollständige Verdauung von DNA durch eine Restriktionsendonuklease. Die Reaktionsbedingungen werden dabei so gewählt, daß in einem DNA-Substrat zwar an einigen, nicht aber an allen Erkennungsstellen für die eingesetzte Restriktionsendonuklease geschnitten wird.

Zur Gewinnung und Isolierung von bestimmten DNA-Fragmenten, z.B. nach Behandlung von DNA mit Restriktionsendonukleasen, wurden die angefallenen DNA-Fragemente in an sich bekannter Weise durch Gelelektrophorese (z.B. auf Agarosegel) getrennt, nachfolgend über das Molekulargewicht (Bestimmung durch Vergleich mit Referenz-DNA-Fragmenten mit bekanntem Molekulargewicht) identifiziert und das gewünschte DNA-Fragment aus der entsprechenden Gelzone abgetrennt.

Ligation (ligieren) bedeutet ein Verfahren zur Bildung von Phosphodiesterbindungen zwischen DNA-Fragmenten (siehe z.B. Maniatis et al., S. 146). Ligationen können unter an sich bekannten Bedingungen, z.B. in einem Puffer mit etwa 10 Units T4-DNA-Ligase pro 0,5 µg der zu ligierenden DNA-Fragmente, ausgeführt werden.

Unter Transformation wird die Einschleusung von DNA in einen Mikroorganismus verstanden, so daß die DNA in diesem repliziert bzw. exprimiert werden kann. Für die Transformation von E. coli ist z.B. die Calciumchloridmethode nach Mandel et al. (1970, J. Mol. Biol. 53: 159) oder nach Maniatis et al. (S. 250 bis 251) geeignet. Für Bacillus-Spezies ist z.B. die Methode Anagnostopoulos etal. (1961, J. Bact. 81: 741 - 746) geeignet.

Bei der Angabe von Enzymstabilitäten in den folgenden Beispielen bedeuten: "völlig stabil" eine Restaktivität von mindestens 90 %; "stabil" eine Restaktivität von wenigstens 80 %.

Die im Beispiel 1 isolierten Bacillus-Stämme sind bei der Deutschen Sammlung von Mikroorganismen und Zellkulturen GmbH (DSM), Bundesrepublik Deutschland (Anschrift: Mascheroder Weg 1B, D-3300 Braunschweig), unter den DSM-Nummern 5776, 5777 bzw. 5778 am 07.02.1990 hinterlegt worden.

### Beispiel 1

Isolierung von Lipase ausscheidenden Bacilli.
A) MMII-Medium (2 % Olivenöl, 0,05 % Hefeextrakt, 0,1 % NaCl, 0,5 % (NH₄)₂SO₄, MgSO₄x2H₂O, 0,2 % Harnstoff, 10 mM Natriumcarbonatpuffer pH 9) wurde mit einer Lebensmittelprobe (Frischkäse, eine Woche bei Raumtemperatur gelagert) beimpft und für 72 Stunden bei 37 °C unter Schütteln inkubiert. Vegetative Zellen wurde durch 30 minütige Behandlung bei 80 °C abgetötet. Verschiedene Verdünnungen der so behandelten Kultur wurden auf Lipase-Screeningplatten, die 2,5 % Olivenöl, 0,8 % Nutrient Broth, 0,4 % NaCl, 2 % Agar, 0,001 % Rhodamin B und 10 mM Natriumcarbonatpuffer (pH 9) enthielten (modifiziert nach Kouker und Jäger, 1987, Appl. Environ. Microbiol. 53, Seiten 211 - 213), ausgespatelt. Die Platten wurden bei 37 °C inkubiert. Nach einer Inkubationsdauer von 2 Tagen wurden um einige Kolonien unter UV-Licht orangefarbige Höfe sichtbar. Eine dieser Kolonien wurde isoliert und der isolierte Stamm wurde bei der Deutschen Sammlung von Mikroorganismen unter der Nummer DSM 5776 hinterlegt.
B) Eine Woche bei Raumtemperatur gelagerte Butter wurde pasteurisiert (30 min, 80 °C). Mit einer Probe hiervon wurde VMI-Medium (1 % Tween 80, 1 % Trypton, 0,5 % Hefeextrakt, 0,5 % NaCl, 10 mM Natriumcarbonatpuffer pH 9) beimpft und für 48 Stunden bei 37 °C unter Schütteln inkubiert. Verschiedene Verdünnungen der Kultur wurden auf Lipase-Screeningplatten ausgespatelt. Nach einer Inkubationsdauer von 2 Tagen wurde um eine Kolonie unter UV-Licht ein orangefarbiger Hof sichtbar. Diese Kolonie wurde isoliert und der isolierte Stamm wurde bei der Deutschen Sammlung von Mikroorganismen unter der Nummer DSM 5777 hinterlegt.
C) Vier Tage bei Raumtemperatur gelagertes Schweineschmalz wurde pasteurisiert (30 min, 80 °C). Mit einer Probe hiervon wurde VMII-Medium (1 % Tween 80, 1 % Olivenöl, 1 % Trypton, 0,5 % Hefeextrakt, 0,5 % NaCl, 10 mM Natriumcarbonatpuffer pH 9) beimpft und für 48 Stunden bei 37 °C unter Schütteln inkubiert. Verschiedene Verdünnungen der Kultur wurden auf Lipase-Screeningplatten ausgespatelt. Nach einer Inkubationsdauer von 2 Tagen wurden um einige Kolonien unter UV-Licht orangefarbige Höfe sichtbar. Eine dieser Kolonien wurde isoliert und der isolierte Stamm wurde bei der Deutschen Sammlung von Mikroorganismen unter der Nummer DSM 5778 hinterlegt.

### Beispiel 2

### Identifizierung der gemäß Beispiel 1 isolierten Bacilli

Es handelt sich bei den in Beispiel 1 isolierten Stämmen mit den DSM-Nummern 5776, 5777 und 5778 um grampositive, sporenbildende aerobe Mikroorganismen, die der Gattung Bacillus zugeordnet werden können. Die zell- und koloniemorphologische Beschreibung wird im folgenden gegeben, biochemiche Reaktionen und Reaktionen auf bestimmte Wuchsbedingungen sind in der Tabelle 1 aufgeführt.

### Bacillus-Spezies DSM 5776:

Stäbchenförmiges Bakterium mit abgerundeten Ecken. Die Gramreaktion (Cramfärbung, KOH-Test) ist positiv. Auf TY-Agar (siehe unten) haben die Kolonien nach 2 Tagen bei 37 °C einen Durchmesser von 3,5 bis 4 mm, sind beigefarben und haben einen glatten bis wellenförmigen Rand. Gelegentlich ist auf den Kolonien auch Tröpfchenbildung zu beobachten; die Kolonien können glänzend oder eingetrocknet-runzlig sein. Die Zellen haben auf TY-Agar eine Größe von 0,7 bis 0,9 µm * 1,2 bis 2,8 um und liegen in der Regel als einzelne Zellen oder in Zweier- oder Dreierketten vor. Die Sporen sind oval und zentral bis subterminal gelegen. Der Stamm sporuliert bereitwillig.

### Bacillus-Spezies DSM 5777:

Stäbchenförmiges mit abgerundeten Ecken. Die Gramreaktion (Gramfärbung, KOH-Test) ist positiv. Auf TY-Agar (siehe unten) haben die Kolonien nach 2 Tagen bei 37 °C einen Durchmesser von 3,2 bis 4,2 mm, sind beigefarben und haben einen glatten bis wellenförmigen Rand. Gelegentlich ist auf den Kolonien auch Tröpfchenbildung zu beobachten; die Kolonien können glänzend oder eingetrocknet-runzlig sein. Die Zellen haben auf TY-Agar eine Größe von 0,8 bis 0,9 µm * 1,2 bis 2,8 um und liegen in der Regel als einzelne Zellen oder in Zweier- oder Dreierketten vor. Die Sporen sind oval und zentral bis subterminal gelegen. Der Stamm sporuliert bereitwillig.

### Bacillus-Spezies DSM 5778:

Stäbchenförmiges Bakterium mit abgerundeten Ecken. Die Gramreaktion (Gramfärbung, KOH-Test) ist positiv. Auf TY-Agar (siehe unten) haben die Kolonien nach 2 Tagen bei 37 °C einen Durchmesser von 2,2 bis 3 mm, sind beigefarben und haben einen glatten bis wellenförmigen Rand. Gelegentlich ist auf den Kolonien auch Tröpfchenbildung zu beobachten; die Kolonien können glänzend oder eingetrocknet-runzlig sein. Die Zellen haben auf TY-Agar eine Größe von 0,7 bis 0,9 µm * 1,3 bis 3,7 µm und liegen in der Regel als einzelne Zellen oder in Zweier- oder Dreierketten vor. Die Sporen sind oval und zentral bis subterminal gelegen. Der Stamm sporuliert bereitwillig.

### TY-Agar:

| | |
|---|---|
| Hefeextrakt | 5 g |
| MgCL₂∗6H₂O | 8,75 g |
| MnCl₂∗6H₂O | 0,016 g |
| Agar | 16 g |
| Aqua bidest. | ad 1000 ml |
| pH | 7,0 ± 0,3 |

Aufgrund der nach Durchführung der in der Tabelle 1 genannten Tests (nach Bergeys Manual of Determinative Bacteriology, Vol.2, 1121-1125, P.H.A. Sneath (ed.), Williams und Wilins, Baltimore-London-Los Angeles-Sydney, 1986) gefundenen Resultate kann eine Zuordnung zu der Art Bacillus pumilus vorgenommen werden. Die isolierten Stämme weichen lediglich in wenigen Merkmalen (Voges-Proskauer Test, pH der V-P Nährlösung und Bildung von Eigelb-Lecithinase, sowie Stamm DSM 5777 hinsichtlich des Wachstums bei 7 % NaCl) von den dort für Bacillus pumilus aufgeführten Kennzeichen ab.

Bacillus pumilus ist ein ubiquitär vorkommender Organismus, der auf Nährboden Kolonien mit variablem Aussehen bildet. Die gemäß Beispiel 1 isolierten Stämme sind-demzufolge:
Bacillus pumilus DSM 5776 (Beispiel 1A),
Bacillus pumilus DSM 5777 (Beispiel 1B) und
Bacillus pumilus DSM 5778 (Beispiel 1C).

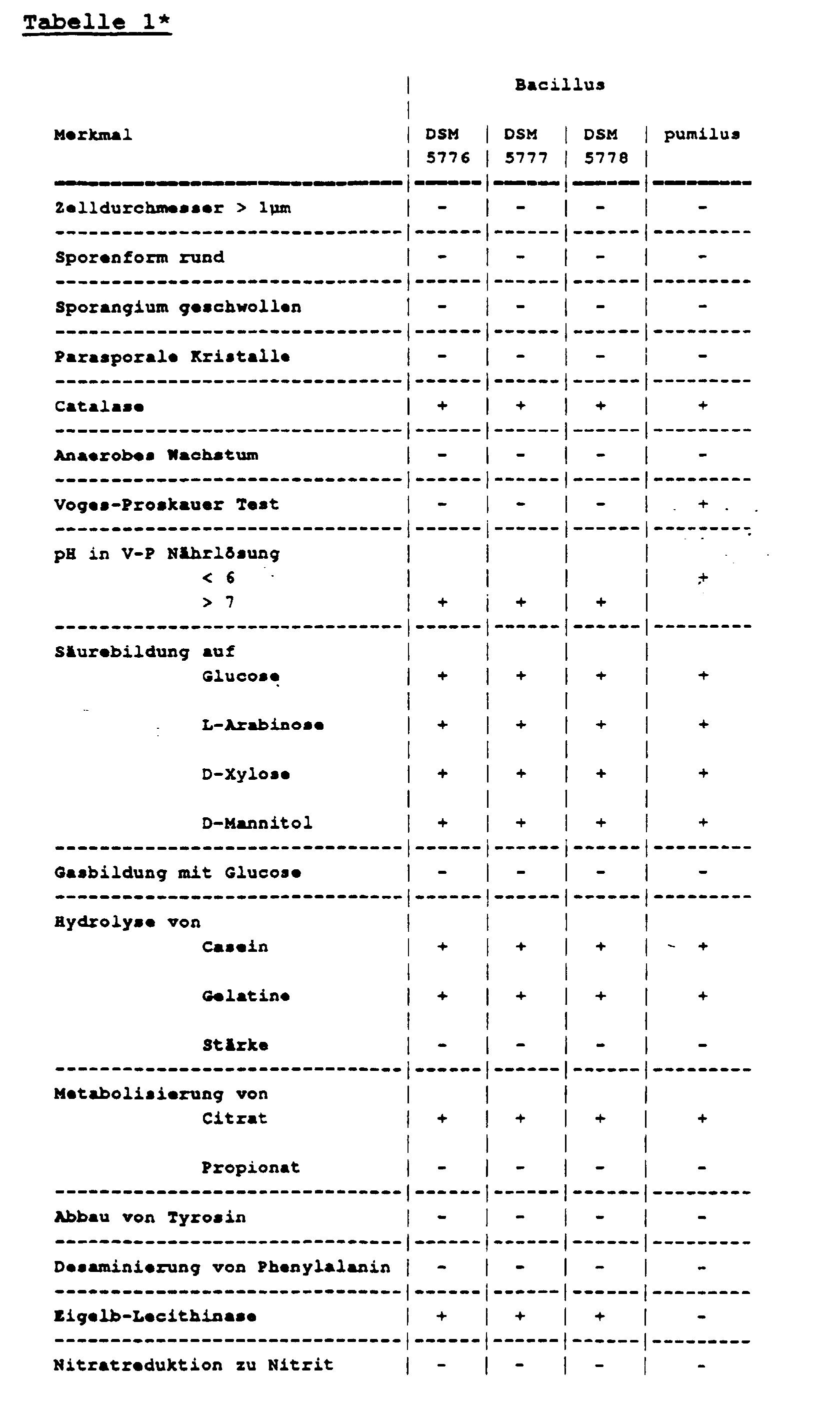

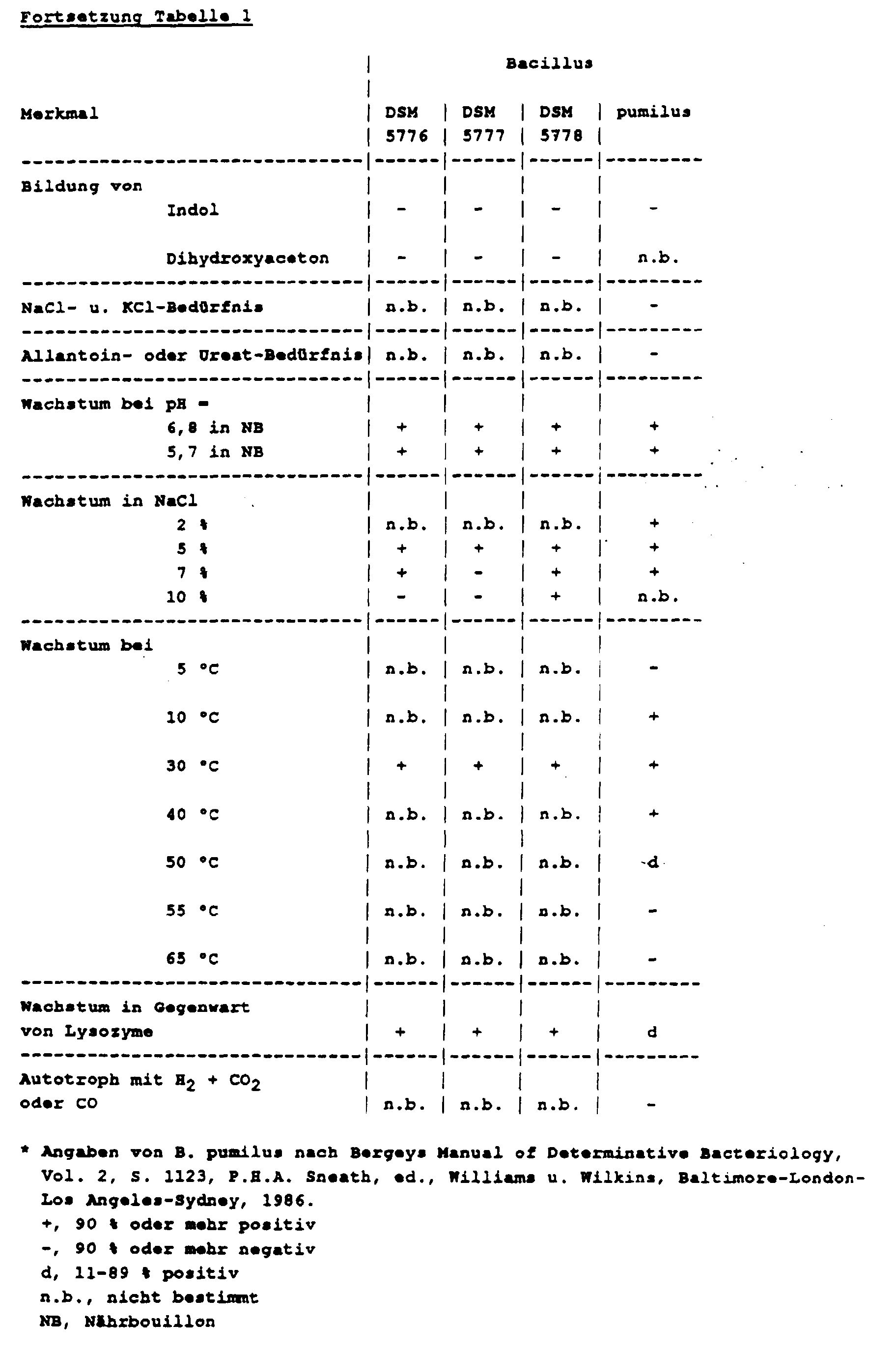

### Beispiel 3

Lipasebildung mit den Bacillus-Naturisolaten des Beispiels 1.

Die im Beispiel 1 isolierten und nach Beispiel 2 näher identifizierten Bacillus-Stämme DSM 5776, DSM 5777 und DSM 5778 wurden bei pH 9,0 und 30 °C bei 300 Upm in einem Medium der nachfolgend angegebenen Zusammensetzung inkubiert.

Zusammensetzung des Mediums: 20 g Sojamehl, 10 g Pepton; 10 g lösliche Stärke; 2 g Dikaliumhydrogenphosphat; 1 g Magnesiumsulfat-heptahydrat; 5,88 g Natriumhydrogencarbonat; 3,18 g Natriumcarbonat; -10 g Tween 80; Aqua bidest. ad 1.000 ml; der pH des komletten Mediums betrug 9,0 ± 0,1.

Nach einer Inkubationsdauer von 22 h für die Bacillus-Stämme DSM 5777 und 5778 bzw. von 41,5 h für den Bacillus-Stamm DSM 5776 wurden die Kulturen durch 15-minütige Zentrifugation von Zellmaterial befreit und die Aktivität der in den Kulturüberständen enthaltenen Lipase gemäß Beispiel 8 ermittelt. Hierbei wurden die nachfolgenden Aktivitäten gemessen, die zeigen, daß die gemäß Beispiel 1 isolierten Bacilli Lipasen ausscheiden.

| Lipase aus Bacillus | Aktivität |
|---|---|
| DSM 5776 | 8,5 U/ml |
| DSM 5777 | 10,3 U/ml |
| DSM 5778 | 12,8 U/ml |

### Beispiel 4

Herstellung genomischer DNA-Bibliotheken aus Bacillus-Naturisolaten und Isolierung von Genen alkalischer Bacillus-Lipasen.

Aus den Naturisolaten Bacillus DSM 5776, Bacillus DSM 5777 und Bacillus DSM 5778 des Beispiels 1 wurde nach der Methode von Saito et al. (1963, Biochim. Biophys. Acta. 72, Seiten 619 - 629) jeweils die chromosomale DNA isoliert und mit der Restriktionsendonuklease Sau3A partiell hydrolisiert. Die Restriktionsfragmente wurden durch Elektrophorese auf einem Agarosegel aufgetrennt, und die Fragmente mit einer Größe von 3 bis 8 Kilobasen (KB) wurden isoliert.

Die isolierten und größenselektierten DNA-Fragmente aus den Bacilli DSM 5776, DSM 5777 und DSM 5778 wurden jeweils mit Vektor-DNA des Plasmids pUB110 (Herstellung wie in Beispiel 7 beschrieben) in vitro neukombiniert.

Hierzu wurde das Plasmid pUB110 zunächst mit der Restriktionsendonuklease BamHI restringiert und anschließend mit alkalischer Phosphatase aus Kälberda dephosphoryliert. Anschließend wurden je 4 µg der restringierten und dephosphorylierten Vektor-DNA mit je 20 µg der DNA-Fragmente aus den Bacilli DSM 5776, DSM 5777 oder DSM 5778 in einem Gesamtvolumen von 200 µl mit T4-DNA Ligase 24 h bei 16 °C inkubiert.

Mit der jeweils erhaltenen in vitro neukombinierten DNA wurden Protoplasten des Stammes Bacillus subtilis PSL 1 (Bacillus Genetic Stock Center 1 A 510) nach der von S. Chang und N. Cohen (1979, Mol. Gen. Genet 168, Seiten 111 - 115) beschriebenen Methode transformiert. Die Transformanten wurden auf Platten mit Neomycin selektiert und anschließend auf Lipase-Screeningplatten (ohne Natriumcarbonat-Puffer) überführt. Unter ca. 100.000 erhaltenen Transformanten wurden einige gefunden, die aufgrund fluoreszierender Höfe um die jeweilige Kolonie als Lipaseausscheider identifiziert werden konnten.

Aus diesen Klonen wurde die jeweilige Plasmid-DNA nach Maniatis et al. isoliert. Das in diesen Plasmiden jeweils enthaltene klonierte Fragment aus Bacillus DSM 5776, Bacillus DSM 5777 oder Bacillus DSM 5778 enthielt (wie durch Beispiel 5 nachgewiesen wurde) die vollständige korrekte DNA-Sequenz für die jeweilige alkalische Bacillus-Lipase.Die Plasmide erhielten die Bezeichnungen pL2-22-11 für das Plasmid mit einem Insert aus der DNAdes Bacillus-Stammes DSM 5776; pL4-22-14 für das Plasmid mit einem Insert aus der DNA des Bacillus-Stammes DSM 5777; pL11-8-20 für das Plasmid mit einem Insert aus der DNAdes Bacillus-Stammes DSM 5778.

Die Plasmide wurden jeweils mit verschiedenen Restriktionsendonukleasen geschnitten, die erhaltenen restringierten DNA's durch Elektrophorese auf einem Agarosegel aufgetrennt und anhand des Bandenmusters vorläufige Restriktionskarten für die Lipasegen-tragenden Inserts in den vorstehend beschriebenen Plasmiden erstellt. Es wurde die jeweils nachfolgend angegebene vorläufige Abfolge von Erkennungsstellen für Restriktionsendonukleasen für die Lipasegen-tragenden Inserts in den oben genannten Plasmiden ermittelt; für das Insert in pL2-22-11 mit einer Größe von etwa 3,5 KB: Clal, Pvull, Ncol, Xbal, Xbal, Acyl, Pvull, Mlul, EcoRV, Bdl, EcoRV; für das Insert in pL4-23-14 mit einer Größe von etwa 2,6 KB: EcoRV, Clal, Ncol, Xbal, Avall, Clal, BgIII, Xbal, EcoRV; für das Insert in pL11-8-20 mit einer Größe von etwa 1,7 KB: Ncol, Avall, Clal, Xbal, EcoRV.

### Beispiel 5

Expression der Lipasegene sowie Überprüfung und Nachweis der lipolytischen Aktivität der exprimierten Bacillus-Lipasen.

Die Plasmide pL2-22-11, pL4-23-14 und pL11-8-20 wurden jeweils erneut in den Stamm B. subtilis PSL 1 eingebracht und die erhaltenen Transformanten kultiviert. Ferner wurde das Plasmid pL2-22-11 in den Bacillus-Stamm DSM 5776, das Plasmid pL4-23-14 in den Bacillus-Stamm DSM 5777 und das Plasmid pL11-8-20 in den Bacillus-Stamm DSM 5778 eingebracht und ebenfalls kultiviert. Als Kontrollstämme wurden ein nichttransformierter B.subtilis PLS 1, ein mit dem Plasmid pUB110 transformierter B. subtilis PSL 1, die Ausgangsstämme für die Isolierung der Lipasegene, die d.h. Bacilli DSM 5776, DSM 5777 und DSM 5778, sowie Transformanten dieserAusgangsstämme mit dem Plasmid pUB 110 ebenfalls kultiviert. Die Transformation erfolgte jeweils nach der im Beispiel 4 angegebenen Methode von S. Chang und N. Cohen. Zur Kultivierung wurden die mit den Plasmiden pL2-22-11, pL4-23-14 oder pL11-8-20 transformierten Bacillus-Stämme und die Kontrollstämme in Schüttelkolben mit 50 ml Vorkultur-Medium (1,5 % Trypton, 1 % Hefeextrakt, 2 % Stärke) 18 Stunden bei 37 °C und 280 Upm inkubiert. Mit 1,5 ml von dieser Kultur wurden Schüttelkolben mit 50 ml Hauptkultur-Medium (1 % Tween 80, 1 % Trypton, 1 % Hefeextrakt, 4 % Stärke, 2 % Sojamehl) beimpft und bei 37 °C und 350 Upm inkubiert. Die Medien für alle plasmidhaltigen Stämme enthielten zusätzlich 10 µg Neomycin/ml. Die Medien für die Bacillus-Stämme DSM 5776, DSM 5777 und DSM 5778 sowie deren plasmidhaltige Abkömmlinge enthielten zusätzlich 10 ml Natriumcarbonat Puffer (1 molar, pH 9,75) pro Liter Medium.

Nach 48 h wurden Proben aus den Kulturen entnommen, zentrifugiert und die lipolytischen Aktivitäten in den Überständen, wie in Beispiel 8 beschrieben, bestimmt. Tabelle 2 zeigt die Ergebnisse dieser Aktivitätsbestimmung.

**Tabelle 2**

| Bacillus-Stämme | Aktivität (U/ml) |
|---|---|
| B. subtilis PSL 1 | <0,1 |
| B. subtilis PSL 1 (pUB110) | <0,1 |
| B. subtilis PSL 1 (pL2-22-11) | 5,3 |
| B. subtilis PSL 1 (pL4-23-14) | 7,2 |
| B. subtilis PSL 1 (pL11-8-20) | 6,4 |
| B. DSM 5776 | 4,8 |
| B. DSM 5776 (pUB110) | 4,5 |
| B. DSM 5776 (pL2-22-11) | 112 |
| B. DSM 5777 | 6,2 |
| B. DSM 5777 (pUB110) | 5,8 |
| B. DSM 5777 (pL4-23-14) | 224 |
| B. DSM 5778 | 5,8 |
| B. DSM 5778 (pUB110) | 5,2 |
| B. DSM 5778 (pL11-8-20) | 153 |

### Beispiel 6

Sequenzierung der Strukturgene für die Bacillus-Lipasen aus den Bacilli mit den DSM-Nummern 5776, 5777 und 5778

Plasmide mit den jeweiligen Lipasegen tragenden Inserts aus der DNA der Bacilli DSM 5776, DSM 5777 und DSM 5778 wurden jeweils mit diversen Restriktionsendonukleasen restringiert. Für jedes der drei Lipasetragenden Inserts wurde so eine Gruppe von Fragmenten erhalten, aus welcher das jeweils kleinste, noch Lipaseaktivität aufweisende Fragment in an sich im Stand der Technik üblicher Weise mit Hilfe von Bacillus subtilis PSL 1 als Wirt subkloniert wurde. Die erhaltenen Plasmide enthielten hierbei jeweils Lipasegen-tragende Insert-Fragmente mit etwa folgender Größenordnung: bei Bacillus DSM 5776 ein etwa 1,45 KB großes DNA-Fragment; bei Bacillus DSM 5777 ein etwa 1,38 KB großes DNA-Fragment; bei Bacillus DSM 5778 ein etwa 1,09 KB großes DNA-Fragment.

Die oben erhaltenen Plasmide mit den Lipasegen-tragenden Fragmenten wurden für die nachfolgend beschriebene Sequenzierung der Strukturgene verwendet. Hierzu wurden aus den jeweiligen Plasmiden durch Schneiden mit Restriktionsendonukleasen die Lipasegen-tragenden Fragmente herausgeschnitten und zur Herstellung von Einzelstrang-DNA in den Phagemiden pBS (+) oder pBS (-) eingebracht; die Phagemide pBS (+/-) wurden von Stratagene (La Jolla, Kalifornien) bezogen. Die Nukleotidsequenzen der in den jeweils isolierten Einzelstrang-Phagemiden enthaltenen Lipasengene wurden nach an sich im Stand der Technik bekannten Methoden, z.B. nach der Dideoxy-Kettenterminator-Methode von Sanger et al. (1977, Proc. Natl. Acad. Sci. USA 74:5463) oder der Methode der basenspezifischen chemischen Spaltung des DNA-Einzelstranges nach Maxam et al. (1980, in Methods in Enzymology, Grossmann L., Modave K., eds., Academic Press Inc., New York und London, Vol. 65, 499) bestimmt. Die für das DNA-Fragment aus Bacillus DSM 5776 ermittelte Nukleotidsequenz und die zugeordnete Aminosäurensequenz der Lipase ist in Fig. 1 wiedergegeben. Der Start für die Aminosäurensequenz der reifen Lipase wurde durch Aminosäurensequenzierung des N-terminalen Endes der Lipase bestimmt. Die DNA-Sequenzen und die angehörigen Aminosäurensequenzen für die Lipasegene aus Bacillus DSM 5777 und DSM 5778 wurden analog bestimmt.

Die Aminosäurensequenz der Lipase aus Bacillus DSM 5777 unterscheidet sich hierbei von der Aminosäurensequenz der Fig. 1 lediglich in Position 149, in der statt Ile die Aminosäure Val steht, und besitzt somit eine Homologie >99 % zur Aminosäurensequenz der Fig. 1.

Die Aminosäurensequenz der Lipase aus Bacillus DSM 5778 unterscheidet sich von der Aminosäurenseqsuenz der Fig. 1 lediglich in 7 Positionen wie folgt: in Postiton 20 steht statt Phe die Aminosäure Tyr; in den Positionen 27-28 stehen statt Ala-Thr die Aminosäuren Val-Gly; in Position 57 und 147 steht statt Arg jeweils die Aminosäure Lys; in den Positionen 149-150 stehen statt Ile-Leu die Aminosäuren Val-Gln. Die Aminosäurensequenz der Lipase aus Bacillus DSM 5778 besitzt somit eine Homologie >96 % zur Aminosäurensequenz der Fig. 1.

### Beispiel 7

Isolierung und Reinigung des Plasmids pUB110.

Aus dem Stamm Bacillus subtilis BD366 (Bacillus Genetic Stock Center 1 E 6) wurde nach der Methode von T.J. Gryczan et al. (1978, J.Bacteriol. 134:318-329) das Plasmid pUB110 isoliert und anschließend nach Maniatis et al. (S. 93) über Cäsiumchlorid-Dichtegradientenzentrifugation gereinigt. Der Vektor pUB110 enthält eine nur einmal vorkommende Restriktionsstelle für die Restriktionsendonuklease BamHI und als Marker eine DNA-Sequenz, die für Antibiotikaresistenz gegenüber Neomycin codiert, sowie für die Replikation in Bacillus-Spezies benötigte DNA-Sequenzen ("origin of replication").

### Beispiel 8

Aktivitätsbestimmung von Bacillus-Lipasen.

Lipasen sind Triacylglycerol-acyl-Hydrolasen der Klasse E.C.3.1. (Klasse nach Enzyme Commission), die emulgierte Triglyceride langkettiger Fettsäuren hydrolysieren. Ort der Lipasewirkung ist die Grenzfläche zwischen Öltröpfchen und der wäßrigen Phase. Hierdurch sind Lipasen eindeutig von Esterasen, die wasserlösliche Substrate umsetzen, unterscheidbar. Zwar können auch Lipasen wasserlösliche Substrate spalten, der Einfluß der Grenzschichtflächen emulgierter Triglyceride auf die Reaktion ist jedoch deutlich. Demzufolge wird die Substratkonzentration für Lipasen statt in mol/l in m²/l angegeben. Der Emulgierungsgrad des Substrates sowie das reproduzierbare Herstellen der Substratemulsion ist von ausschlaggebender Bedeutung für den Lipase-Aktivitätstest. Als Emulgatoren finden beispielsweise insbesondere Gummi arabicum, Polyvinylalkohol oder Natriumdesoxycholat Verwendung.

Das Reaktionsprodukt der Hydrolyse, die freie Fettsäure, wird in der Regel titrimetrisch erfaßt. Aus der Fülle der bekannten titrimetrischen Methoden wurde hier eine direkte kontinuierliche Titration unter pH-Statbedingungen während der Reaktion durchgeführt. Bei der Durchführung dieses kontinuierlichen titrimetrischen Tests ist zu beachten, daß beim Einsatz langkettiger Triglyceride als Substrat die apparente Protolysenkonstante pKₐ langkettiger Triglyceride etwa bei pH = 9 liegt. Werden Messungen bei kleineren pH-Werten durchgeführt, so wird nur ein geringerTeil des gebildeten Produkts titrimetrisch erfaßt. Der apparente pKₐ-Wert kann durch Zugabe von Natriumchlorid beeinflußt werden.

### Beschreibung des Aktivitätstests:

- Substratemulsion: Triolein 10 g; Gummi arabicum 10 g; aqua bidest. 100 ml. Die Emulsion wurde mit einem Labormixer 15 min. bei hoher Drehzahl emulgiert.
- Puffer: Natriumchlorid 100 mM; Calciumchlorid-dihydrat 20 mM.
- Lauge: Natronlauge 10 mM.
- Temperatur: 30 °C.
- pH-Statapparat: bestehend aus pH-Meter, Steuergerät Dosierpumpe und Aufzeichnungsgerät für den Laugenverbrauch.

### Durchführung:

20 ml der Pufferlösung und 10 ml der Substratemulsion wurden zusammengegeben, auf 30 °C erwärmt und der pH-Wert dieses Gemisches auf 9,5 eingestellt. Anschließend wurden 0,2 bis 0,5 ml der Probe bzw. der Blindwertlösung (Zusammensetzung wie die Probe, aber ohne aktive Lipase; Probe vor der Aktivitätsbestimmung thermisch inaktiviert) hinzugegeben und der pH-Wert durch Titration mit Natronlauge konstant bei 9,5 gehalten. Der Laugenverbrauch wurde über die gesamte Versuchsdauer durch ein Aufzeichnungsgerät protokolliert. Eine Unit (U) Lipaseaktivität bewirkt unter den angegebenen Bedingungen die Freisetzung von 1 µmol Fettsäure pro Minute.

### Beispiel 9

Bestimmung der Enzymcharakteristika von Bacillus-Lipasen.

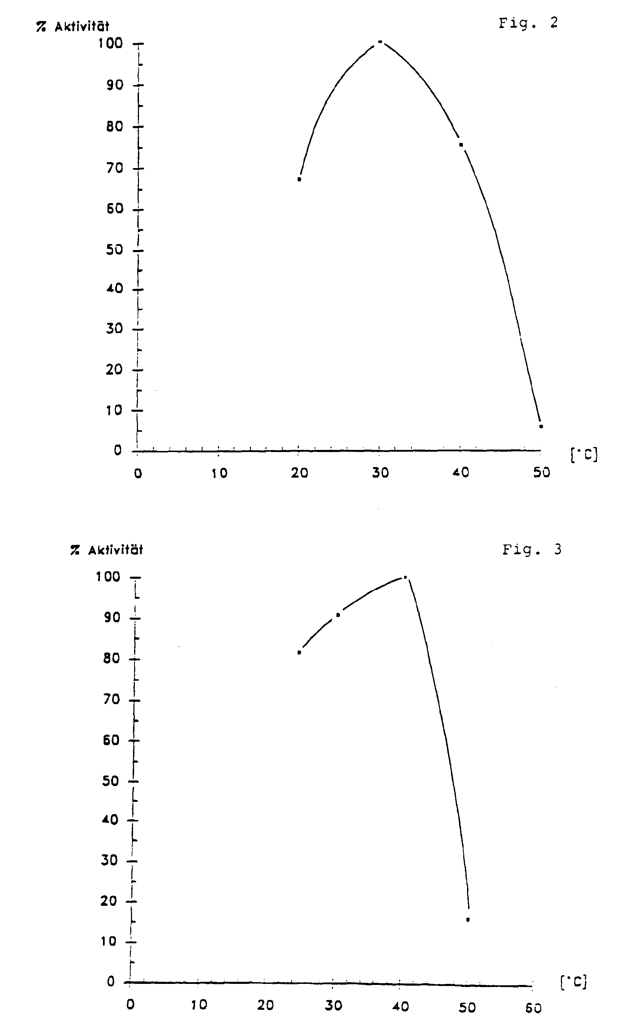

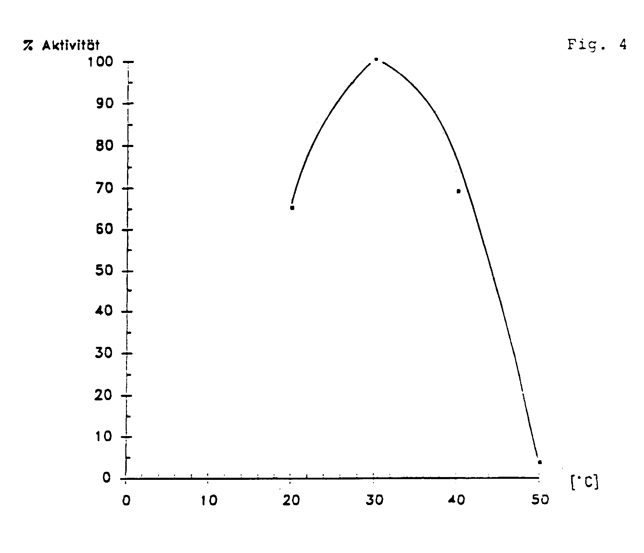

Zur Bestimmung der Enzymcharakteristika wie Temperatur-Optimum, Temperatur-Stabilität, pH-Optimum und pH-Stabilität wurden die im Beispiel 5 durch Kultivierung von Bacilli erhaltenen Kulturüberstände für 30 Minuten bei 100.000-facher Erdbeschleunigung zentrifugiert, um im Kulturüberstand vorhandene Zellen der Bacilli abzuscheiden. Die durch Zentrifugation erhaltene, klare überstehende Lösung wurde für die nachfolgenden, unter A) bis D) beschriebenen Messungen eingesetzt.
A) Das Temperatur-Optimum der in den Kulturüberständen enthaltenen Lipasen wurde mit Hilfe des in Beispiel 8 beschriebenen Aktivitätstest bestimmt. Dazu wurde die Temperatur im Bereich von 20 bis 50 °C variiert. Die Ergebnisse sind in Tabelle 3 sowie in den Figuren 2, 3 bzw. 4 dargestellt.
Das Temperatur-Optimum der Lipase aus Bacillus
DSM 5776 liegt bei 30 °C (Fig. 2).
Das Temperatur-Optimum der Lipase aus Bacillus
DSM 5777 liegt bei etwa 40 °C (Fig. 3).
Das Temperatur-Optimum der Lipase aus Bacillus
DSM 5778 liegt bei 30 °C (Fig. 4).

**Tabelle 3**

| alkalische Lipase aus Bacillus | Lipaseaktivität in % in Abhängigkeit von der Temperatur in °C | | | | |
|---|---|---|---|---|---|
| | 20 | 24 | 30 | 40 | 50 |
| DSM 5776 | 66,8 | - | 100 | 74,9 | 5,3 |
| DSM 5777 | - | 81,2 | 90,4 | 100 | 15,9 |
| DSM 5778 | 64,7 | - | 100 | 68,4 | 3,2 |

B) Zur Bestimmung der Temperatur-Stabilität wurden die Lipase-haltigen Überstände für 30 Minuten bei verschiedenen Temperaturen inkubiert und anschließend die Restaktivität nach der in Beispiel 8 angegebenen Methode zur Aktivitätsbestimmung bestimmt. Die Ergebnisse sind in der Tabelle 4 sowie in den Figuren 5, 6 und 7 dargestellt.
Die Lipase aus Bacillus DSM 5776 ist bis 40 °C stabil und zeigt nach 30 minütiger Inkubation bei 50 °C noch eine Restaktivität von 16,1 % (Fig. 5).
Es zeigte sich, daß die Lipase aus Bacillus DSM 5777 bis 40 °C stabil ist und bei 50 °C noch eine Restaktivität von 21,3 % besitzt (Fig. 6).
Die Lipase aus Bacillus DSM 5778 ist bis 40 °C stabil und zeigt nach 30 minütiger Inkubation bei 50 °C noch eine Restaktivität von 22,5 % (Fig. 7).

**Tabelle 4**

| alkalische Lipase aus Bacillus | Lipase-Restaktivität in % in Abhängigkeit von der Inkubations-Temperatur in °C | | | | |
|---|---|---|---|---|---|
| | 30 | 40 | 50 | 60 | 70 |
| DSM 5776 | 105 | 100 | 16 | 2 | 2 |
| DSM 5777 | 96 | 102 | 22 | 9 | - |
| DSM 5778 | 95 | 90 | 24 | 0 | - |

C) Zur Bestimmung der pH-Optima der Bacillus-Lipasen wurde die in Beispiel 8 angegebene Aktivitätsbestimmung bei verschiedenen pH-Werten durchgeführt. Aufgrund der bei pH-Werten < 9 schwierigen Erfassung des Produktes der enzymatischen Umsetzung, wurde hier allerdings nicht kontinuierlich, sondern erst nach Ablauf der Reaktionszeit auf pH = 9,5 titriert. Die Ergebnisse sind in der Tabelle 5 sowie in den Figuren 8, 9 und 10 dargestellt.
Das pH-Optimum der alkalischen Lipase aus Bacillus
DSM 5776 liegt bei pH = 9,5. Bei pH = 10 ist die Aktivität noch > 90 % (Fig. 8).
Das pH-Optimum der alkalischen Lipase aus Bacillus
DSM 5777 liegt bei etwa pH = 10 (Fig. 10).
Das pH-Optimum der alkalischen Lipase aus Bacillus
DSM 5778 liegt bei pH = 9, aber auch bei pH = 10 sind noch etwa 80 % der maximalen Aktivität vorhanden (Fig. 10).

**Tabelle 5**

| alkalische Lipase aus Bacillus | Lipaseaktivität in % in Abhängigkeit vom pH-Wert | | | | | | | | |
|---|---|---|---|---|---|---|---|---|---|
| | 5 | 6 | 7 | 7,8 | 8 | 9 | 9,5 | 10 | 10,5 |
| DSM 5776 | - | 53 | 65 | 76 | - | 95 | 100 | 95 | 55 |
| DSM 5777 | 30 | 56,8 | 76 | - | 88 | 92,2 | 99,8 | 100 | 78 |
| DSM 5778 | 25 | 53 | 73 | - | 88 | 100 | 93,5 | 80 | 40 |

D) Zur Untersuchung der pH-Stabilität wurden die Lipasen für 21 h in Puffern verschiedenen pH-Wertes bei 4 °C inkubiert. Anschließend wurde die Restaktivität der Lipasen, wie unter Beispiel 8 beschrieben, bestimmt. Für den pH-Bereich von 5 bis 7 wurde Phosphat-Puffer, für den pH-Bereich von 7 bis 9 Tris-HCl-Puffer (= Tris (hydroxymethyl)aminomethan-Puffer) und für den pH-Bereich von 9 bis 12,8 Glycin/Natriumhydroxid-Puffer verwendet. Die Ergebnisse sind In den Figuren 11, 12 und 13 dargestellt.
Das Enzym aus Bacillus DSM 5776 ist im pH-Bereich von 5 bis 11 völlig stabil und nach 21-stündiger Inkubation bei pH = 12 ist noch eine Restaktivität von 22 % vorhanden (Fig. 11).
Die Lipase aus Bacillus DSM 5777 ist im pH-Bereich von 6 bis 11 stabil (Fig. 12).
Die Lipase aus Bacillus DSM 5778 ist im pH-Bereich von 6,5 bis 11,5 völlig stabil (Fig. 13).

### Beispiel 10

### Waschversuche

Die Waschwirksamkeit der Bacillus-Lipasen aus den Bacilli DSM 5776, DSM 5777 und DSM 5778 wurde bei niedrigen Waschtemperaturen in einer Standardwaschmittelzusammensetzung durch Waschversuche untersucht. Die Ergebnisse zeigen, daß die erfindungsgemäßen Bacillus-Lipasen auch bei niedrigen Waschtemperaturen, bei denen übliche Waschmittel kaum Ölbzw. Fettverschmutzungen von der Wäsche entfernen, hervorragend für den Einsatz in Waschmitteln geeignet sind.

### A) Testgewebe

Die Eignung von Enzymen für den Einsatz in Wachmitteln wird im allgemeinen durch Waschtests geprüft. Dafür sind spezielle Testgewebe käuflich erhältlich, z.B. von der Eidgenössischen Materialprüfungs- und Versuchsanstalt in St. Gallen (Schweiz), EMPA Die von EMPA vertriebenen Gewebe sind jedoch in erster Linie für den Test von Waschmittelproteasen gedacht. Für den Test von Waschmittellipasen müssen daher speziell präparierte Testgewebe eigens hergestellt werden, z.B. indem das Testgewebe teilweise mit Öl oder mit einer Mischung aus Pigmenten, Proteinen und Öl beschmutzt wird oder auch das ganze Testgewebe mit Öl, beispielsweise Olivenöl, imprägniert wird.

Für die nachfolgenden Waschversuche wurde Baumwollgewebe in Olivenöl (extra vergine) getränkt und dann von überschüssigem Öl befreit. Anschließend wurde das Gewebe bei 30 °C getrocknet. Um das Altern der Ölverschmutzungen zu inhibieren wurde das derart erhaltene, mit Öl verschmutzte Gewebe unter eine Stickstoffatmosphäre bei 4 °C bis zum Gebrauch aufbewahrt.

### B) Durchführung der Waschversuche

Zur Durchführung der Waschtests wurden die unter A) dieses Beispiels hergestellten Testgewebe in Stücke geeigneter Größe, z.B. 5 cm x 5 cm, zerschnitten. Diese Testläppchen wurden in einer Linitest-Waschmaschine mitje 100 ml Waschlauge aus Perborat-haltigem IEC-Testwaschmittel (Konzentration 6 g/l) der nachfolgenden Zusammensetzung bei einer Temperatur von 40 °C für 30 Minuten gewaschen.

Zusammensetzung des Perborat-haltigen IEC-Testwaschmittels, Type 1 (Lever Sunlicht GmbH, Mannheim): 6,4 Gew.-% lineare Alkylsulfonate, 2,3 Gew.-% Ethoxylierte Fettalkohole mit 14 % Ethoxygruppen, 2,8 Gew.-% Natriumseife, 35 Gew.-% Natriumtripolyphosphat, 6 Gew.-% Natriumsilikat, 1,5 Gew.-% Magnesiumsilikat, 1 Gew.-% Carboxymethylcellulose, 0,2 Gew.-% Ethylendiamintetraessigsäure (EDTA), 0,2 Gew.-% optische Aufheller (Stilbentyp), 16,8 Gew.-% Natriumsulfat und 7,8 Gew.-% Wasser, als sprühgetrocknetes Pulver ohne Bleichaktivator sowie mit 20 Gew.-% Natriumperborat-Tetrahydrat.

Es wurde in Wasser mit 15 °dH (Grad deutscher Härte) gewaschen. Anschließend wurde das Gewebe einem Spülvorgang mit Leitungswasser unterworfen. Es wurden jeweils 5 solcher Waschzyklen durchgeführt. Nach Beendigung des gesamten Waschvorganges wurden die Testläppchen erneut mit Leitungswasser gespült, getrocknet und anschließend das noch anhaftende Restöl mit Chloroform extrahiert. Das auf dieses Weise gewonnene Restöl wurde gravimetrisch bestimmt. Durch dieses Methode lassen sich Unterschiede zwischen den Lipasen, d.h. ob diese nicht, nur mäßig oder gut für die Anwendung in Waschmitteln geeignet sind, hervorragend ermitteln. Zu einem Teil der Waschlaugen wurde zusätzlich eine alkalische Waschmittelprotease zugegeben, um so gleichzeitig die Waschwirksamkeit und die Stabilität der Bacillus-Lipasen in Gegenwart der Protease aufzuzeigen. Die Aktivität der eingesetzten Protease in der Waschlauge betrug jeweils 5 DU/ml (DU = Delft Units). Die jeweils eingesetzte Lipaseaktivität in der Waschlauge ist nachfolgend im Zusammenhang mit den Ergebnissen angegeben.

Waschversuche mit der alkalischen Bacillus-Lipase aus Bacillus DSM 5776 mit einer Lipase-Konzentration in der Waschlauge von 60 U/ml (siehe auch Fig. 14):

| Waschlauge | Restöl in Gew.-% |
|---|---|
| IEC-Testwaschmittel (Kontrolle) | 100 |
| IEC-Testwaschmittel + Lipase | 54,5 |
| IEC-Testwaschmittel + Lipase + alkalische Protease | 57,0 |

Waschversuche mit der alkalischen Bacillus-Lipase aus Bacillus DSM 5777 mit einer Lipase-Konzentration in der Waschlauge von 45 U/ml (siehe auch Fig. 15):

| Waschlauge | Restöl in Gew.-% |
|---|---|
| IEC-Testwaschmittel (Kontrolle) | 100 |
| IEC-Testwaschmittel + Lipase | 44,9 |
| IEC-Testwaschmittel + Lipase + alkalische Protease | 50,0 |

Waschversuche mit der alkalischen Bacillus-Lipase aus Bacillus DSM 5778 mit einer Lipase-Konzentration in der Waschlauge von 55 U/ml (siehe auch Fig. 16):

| Waschlauge | Restöl in Gew.-% |
|---|---|
| IEC-Testwaschmittel (Kontrolle) | 100 |
| IEC-Testwaschmittel + Lipase | 54,5 |
| IEC-Testwaschmittel + Lipase + alkalische Protease | 57,0 |

Die Ergebnisse zeigen die hervorragende Eignung der erfindungsgemäßen Bacillus-Lipasen für den Einsatz in Waschmitteln. Die erfindungsgemäßen Lipasen senken den Restölgehalt gegenüber der Kontrolle erheblich und werden auch in Anwesenheit einer alkalischen Waschmittelprotease in ihrer Waschwirksamkeit nicht negativ beeinflußt.

### Beispiel 11

Stabilität der Bacillus-Lipasen in Waschlauge mit und ohne Zusatz von alkalischer Waschmittelprotease.

Zum Nachweis der Stabilität der erfindungsgemäßen Lipasen in Waschlauge mit und ohne Zusatz von alkalischen Waschmittelproteasen wurden Waschversuche wie in Beispiel 10 durchgeführt. Zu Beginn des Waschprozesses (t = 0) und zum Ende des Waschprozesses (t = 30 min) wurde die Aktivität der Lipase analog zu der im Beispiel 8 angegebenen Methode bestimmt. Die relative Restaktivität der Lipasen nach Abschluß des Waschprozesses, bezogen auf die zum Zeitpunkt t = 0 eingesetzte Anfangsaktivität der Lipasen, wird nachfolgend angegeben.

| Waschlauge | relative Restaktivität in % |
|---|---|
| Lipase aus Bacillus DSM 5776 | 50,5 |
| Lipase aus Bacillus DSM 5776 + alkalische Protease | 47,0 |
| | |
| Lipase aus Bacillus DSM 5777 | 69,4 |
| Lipase aus Bacillus DSM 5777 + alkalische Protease | 65,5 |
| | |
| Lipase aus Bacillus DSM 5778 | 49,9 |
| Lipase aus Bacillus DSM 5778 + alkalische Protease | 42,3 |

Die Ergebnisse zeigen, daß die erfindungsgemäßen alkalischen Bacillus-Lipasen in Waschlaugen, auch in Gegenwart von Proteasen, sehr gute Stabilitäten besitzen. Die erfindungsgemäßen Lipasen eignen sich daher sehr gut für die Verwendung in Waschmittelzusammensetzungen, insbesondere auch in Protease-haltigen Waschmittelzusammensetzungen.

## Patentansprüche (Patentansprüche für folgende(n) Vertragsstaat(en): AT, BE, CH, DE, DK, FR, GB, GR, IT, LI, LU, LN, SE)

1. Bacillus-Lipasen mit einem pH-Optimum im alkalischen pH-Bereich und einem Temperatur-Optimum im Bereich von etwa 30 bis 40 °C, erhältlich durch Kultivierung von Bacillus pumilus DSM 5776, DSM 5777 oder DSM 5778.

2. Alkalische Bacillus-Lipasen nach Anspruch 1, dadurch gekennzeichnet, daß sie eine Aminosäurensequenz besitzen, die wenigstens 70 % Homologie zu der in Fig. 1 angegebenen Aminosäurensequenz aufweist.

3. Alkalische Bacillus-Lipasen nach Anspruch 2, dadurch gekennzeichnet, daß deren Aminosäurensequenz wenigstens 80 % Homologie zu der in Fig. 1 angegebenen Aminosäurensequenz aufweist.

4. Alkalische Bacillus-Lipasen nach einem der Ansprüche 1 bis 3 mit folgenden Eigenschaften:
(1) Wirkung: Abbau von Triacylglyceriden und von Fettsäureestern;
(2) pH-Optimum: etwa bei pH-Werten von 9 bis 10;
(3) pH-Stabilität: bei pH-Werten von 6,5 bis 11 erweisen sich die Enzyme als völlig stabil; nach Inkubation bei pH 11 und 4 °C für 21 h beträgt die Restaktivität der Enzyme wenigstens 90 %;
(4) Temperatur-Optimum: etwa 30 bis 40 °C;
(5) Temperatur-Stabilität: durch Inkubation der Enzyme bei Temperaturen bis 40 °C für30 Minuten werden die Enzyme nicht wesentlich in ihrer Aktivität beeinträchtigt; nach 30 Minuten Inkubation bei 40 °C beträgt die Restaktivität der Enzyme wenigstens 90 %.

5. DNA-Sequenz, codierend für eine alkalische Bacillus-Lipase mit einer Aminosäurensequenz, die wenigstens 70 % Homologie zu der in Fig. 1 angegebenen Aminosäurensequenz aufweist.

6. DNA-Sequenz nach Anspruch 5, dadurch gekennzeichnet, daß sie für eine Aminosäurensequenz codiert, die wenigstens 80 %, vorzugsweise wenigstens 90 %, Homologie zu der in Fig. 1 angegebenen Aminosäurensequenz aufweist.

7. Für die Transformation von Mikroorganismen geeigneter Vektor, vorzugsweise ein Expressionsvektor, enthaltend eine DNA-Sequenz gemäß Anspruch 5 oder 6.

8. Zur Expression von Bacillus-Lipasen geeignete, transformierte Mikroorganismen, dadurch gekennzeichnet, daß sie mit einem Vektor gemäß Anspruch 7 transformiert sind.

9. Transformierter Mikroorganismus nach Anspruch 8, dadurch gekennzeichnet, daß dieser ein transformierter Bacillus subtilis, Bacillus alcalophilus, Bacillus licheniformis oder Bacillus amyloliquefaciens ist.

10. Bacillus pumilus der Spezies DSM 5776, DSM 5777 und DSM 5778.

11. Zusammensetzungen zum Waschen, Reinigen, Bleichen oder Geschirrspülen, enthaltend eine alkalische Bacillus-Lipase gemäß einem der Ansprüche 1 bis 4.

12. Zusammensetzungen nach Anspruch 11, dadurch gekennzeichnet, daß sie die alkalische Bacillus-Lipase in Gegenwart von Protease enthalten.

13. Zusammensetzungen nach Anspruch 11 oder 12, dadurch gekennzeichnet, daß sie die alkalische Bacillus-Lipase in einer Formulierung für niedrige Anwendungstemperaturen, vorzugsweise für Anwendungstemperaturen von etwa 30 bis 40 °C, enthalten.

14. Verfahren zur Herstellung von alkalischen Bacillus-Lipasen gemäß einem der Ansprüche 1 bis 4, dadurch gekennzeichnet, daß man einen Mikroorganismus gemäß einem der Ansprüche 8 und 9 oder einen Bacillus pumilus kultiviert und nachfolgend die gebildete alkalische Bacillus-Lipase isoliert.

15. Verfahren nach Anspruch 14, dadurch gekennzeichnet, daß man einen Bacillus pumi lus der Spezies DSM 5776, DSM 5777 oder DSM 5778 kultiviert.

## Patentansprüche (Patentansprüche für folgende(n) Vertragsstaat(en): ES)

1. Verfahren zur Herstellung von Lipasen mit einem pH-Optimum im alkalischen pH-Bereich und einem Temperatur-Optimum im Bereich von etwa 30 bis 40 °C, dadurch gekennzeichnet, daß man einen Bacillus pumilus oder einen mit einem Expressionsvektor - enthaltend eine DNA-Sequenz, codierend für eine alkalische Bacillus-Lipase mit einer Aminosäurensequenz, die wenigstens 70 % Homologie zu der in Fig. 1 angegebenen Aminosäurensequenz aufweist - transformierten Mikroorganismus, vorzugsweise einen transformierten Bacillus subtilis, Bacillus alcalophilus, Bacillus licheniformis oder Bacillus amyloliquefaciens, kultiviert und nachfolgend die gebildete alkalische Bacillus-Lipase isoliert.

2. Verfahren nach Anspruch 1, dadurch gekennzeichnet, daß man einen Bacillus pumilus der Spezies DSM 5776, DSM 5777 oder DSM 5778 kultiviert.

3. Verfahren zur Herstellung von alkalischen Bacillus-Lipasen nach Anspruch 2, dadurch gekennzeichnet, daß man Lipasen herstellt, die eine Aminosäurensequenz besitzen, die wenigstens 70 % Homologie zu der in Fig. 1 angegebenen Aminosäurensequenz aufweist.

4. Verfahren zur Herstellung von alkalischen Bacillus-Lipasen nach Anspruch 3, dadurch gekennzeichnet, daß man Lipasen herstellt, deren Aminosäurensequenz wenigstens 80 % Homologie zu der in Fig. 1 anangegebenen.Aminosäurensequenz aufweist.

5. Verfahren zur Herstellung von alkalischen Bacillus-Lipasen nach einem der Ansprüche 1 bis 4, dadurch gekennzeichnet, daß man Lipasen mit folgenden Eigenschaften herstellt:
(1) Wirkung: Abbau von Triacylglyceriden und von Fettsäureestern;
(2) pH-Optimum: etwa bei pH-Werten von 9 bis 10;
(3) pH-Stabilität: bei pH-Werten von 6,5 bis 11 erweisen sich die Enzyme als völlig stabil; nach Inkubation bei pH 11 und 4 °C für 21 h beträgt die Restaktivität der Enzyme wenigstens 90 %;
(4) Temperatur-Optimum: etwa 30 bis 40 °C;
(5) Temperatur-Stabilität: durch Inkubation der Enzyme bei Temperaturen bis 40 °C für30 Minuten werden die Enzyme nicht wesentlich in ihrer Aktivität beeinträchtigt; nach 30 Minuten Inkubation bei 40 °C beträgt die Restaktivität der Enzyme wenigstens 90 %.

6. Verfahren zur Herstellung einer DNA-Sequenz, codierend für eine alkalische Bacillus-Lipase mit einer Aminosäurensequenz, die wenigstens 70 % Homologie zu der in Fig. 1 angegebenen Aminosäurensequenz aufweist, durch Kultivierung eines Bacillus-Stammes, der die Lipase mit der angegebenen Aminosäurensequenz produziert und Isolierung der für die Lipase codierenden DNA-Sequenz.

7. Verfahren zur Herstellung einer DNA-Sequenz nach Anspruch 6, dadurch gekennzeichnet, daß man eine DNA-Sequenz herstellt, die für eine Aminosäurensequenz codiert, die wenigstens 80 %, vorzugsweise wenigstens 90 %, Homologie zu der in Fig. 1 angegebenen Aminosäurensequenz aufweist.

8. Verfahren zur Herstellung eines für die Transformation von Mikroorganismen geeigneten Vektors, vorzugsweise eines Expressionsvektors, enthaltend eine DNA-Sequenz gemäß Anspruch 6 oder 7, durch Einbringen der für die Lipase codierenden DNA-Sequenz in eine Vektor-DNA.

9. Verfahren zur Herstellung von zur Expression von Bacillus-Lipasen geeigneten, transformierten Mikroorganismen, dadurch gekennzeichnet, daß man einen Vektor gemäß Anspruch 8 in einen geeigneten Mikroorganismus transformiert.

10. Verfahren zur Herstellung eines transformierten Mikroorganismus nach Anspruch 9, dadurch gekennzeichnet, daß man einen Bacillus subtilis, Bacillus alcalophilus, Bacillus licheniformis oder Bacillus amyloliquefaciens transformiert.

11. Verfahren zur Herstellung eines Bacillus pumilus der Spezies DSM 5776, DSM 5777 und DSM 5778 durch Kultivierung der bei der Deutschen Sammlung von Mikroorganismen, Bundesrepublik Deutschland, hinterlegten Bacillus-Naturisolate mit den Nummern DSM 5776, DSM 5777 oder DSM 5778.

12. Zusammensetzungen zum Waschen, Reinigen, Bleichen oder Geschirrspülen, enthaltend eine alkalische Bacillus-Lipase, die gemäß einem Verfahren der Ansprüche 1 bis 5 erhältlich ist.

13. Zusammensetzungen nach Anspruch 12, dadurch gekennzeichnet, daß sie die alkalische Bacillus-Lipase in Gegenwart von Protease enthalten.

14. Zusammensetzungen nach Anspruch 12 oder 13, dadurch gekennzeichnet, daß sie die alkalische Bacillus-Lipase in einer Formulierung für niedrige Anwendungstemperaturen, vorzugsweise für Anwendungstemperaturen von etwa 30 bis 40 °C, enthalten.

## Claims (Claims for the following Contracting State(s): AT, BE, CH, DE, DK, FR, GB, GR, IT, LI, LU, NL, SE)

1. Bacillus-Lipases with a pH optimum in the alkaline pH range and a temperature optimum in the range from about 30 to 40°C, obtainable by cultivation of Bacillus pumilus DSM 5776, DSM 5777 or DSM 5778.

2. Alkaline Bacillus lipases according to Claim 1, characterised in that they have an aminoacid sequence which displays at least 70% homology with the amino-acid sequence indicated in Fig. 1.

3. Alkaline Bacillus lipases according to Claim 2, characterised in that their amino-acid sequence displays at least 80% homology with the aminoacid sequence indicated in Fig. 1.

4. Alkaline Bacillus lipases according to any of Claims 1 to 3, with the following properties:
(1) action: degradation of triacylglycerides and of fatty acid esters;
(2) pH optimum: approximately at pH values from 9 to 10;
(3) pH stability: at pH values from 6.5 to 11 the enzymes prove to be completely stable; the activity of the enzymes remaining after incubation at pH 11 and 4°C for 21 h is at least 90%;
(4) temperature optimum: about 30 to 40°C;
(5) temperature stability: essentially no impairment of the enzymes' activity by incubation of the enzymes at temperatures up to 40°C for 30 minutes; the activity of the enzymes remaining after incubation at 40°C for 30 minutes is at least 90%.

5. DNA sequence coding for an alkaline Bacillus lipase with an aminoacid sequence which displays at least 70% homology with the amino-acid sequence indicated in Fig. 1.

6. DNA sequence according to Claim 5, characterised in that it codes for an aminoacid sequence which displays at least 80%, preferably at least 90%, homology with the amino-acid sequence indicated in Fig. 1.

7. Vector suitable for the transformation of micro-organisms, preferably an expression vector, containing a DNA sequence according to Claim 5 or 6.

8. Transformed microorganisms suitable for the expression of Bacillus lipases, characterised in that they are transformed with a vector according to Claim 7.

9. Transformed microorganism according to Claim 8, characterised in that the latter is a transformed Bacillus subtilis, Bacillus alcalophilus, Bacillus licheniformis or Bacillus amyloliquefaciens.

10. Bacillus pumilus of the species DSM 5776, DSM 5777 and DSM 5778.

11. Compositions for washing, cleaning, bleaching or dishwashing, containing an alkaline Bacillus lipase according to any of Claims 1 to 4.

12. Compositions according to Claim 11, characterised in that they contain the alkaline Bacillus lipase in the presence of protease.

13. Compositions according to Claim 11 or 12, characterised in that they contain the alkaline Bacillus lipase in a formulation for low use temperatures, preferably for use temperatures from about 30 to 40°C.

14. Process for the preparation of alkaline Bacillus lipases according to any of Claims 1 to 4, characterised in that a microorganism according to either of Claims 8 and 9 or a Bacillus pumilus is cultivated and subsequently the produced alkaline Bacillus lipase is isolated.

15. Process according to Claim 14, characterised in that a Bacillus pumilus of the species DSM 5776, DSM 5777 or DSM 5778 is cultivated.

## Claims (Claims for the following Contracting State(s): ES)

1. Process for the preparation of lipases with a pH optimum in the alkaline pH range and a temperature optimum in the range from about 30 to 40°C, characterised in that a Bacillus pumilus or a microorganism which is transformed with an expression vector - containing a DNA sequence coding for an alkaline Bacillus lipase with an aminoacid sequence which displays at least 70% homology with the amino-acid sequence indicated in Fig. 1 - preferably a transformed Bacillus subtilis, Bacillus alcalophilus, Bacillus licheniformis or Bacillus amyloliquefaciens, is cultivated and subsequently the produced alkaline Bacillus lipase is isolated.

2. Process according to Claim 1, characterised in that a Bacillus pumilus of the species DSM-5776, DSM 5777 or DSM 5778 is cultivated.

3. Process for the preparation of alkaline Bacillus lipases according to Claim 2, characterised in that lipases which have an aminoacid sequence which displays at least 70% homology with the aminoacid sequence indicated in Fig. 1 are prepared.

4. Process for the preparation of alkaline Bacillus lipases according to Claim 3, characterised in that lipases which have an aminoacid sequence which displays at least 80% homology with the aminoacid sequence indicated in Fig. 1 are prepared.

5. Process for the preparation of alkaline Bacillus lipases according to any of Claims 1 to 4, characterised in that lipases with the following properties are prepared:
(1) action: degradation of triacylglycerides and of fatty acid esters;
(2) pH optimum: approximately at pH values from 9 to 10;
(3) pH stability: at pH values from 6.5 to 11 the enzymes prove to be completely stable; the activity of the enzymes remaining after incubation at pH 11 and 4°C for 21 h is at least 90%;
(4) temperature optimum: about 30 to 40°C;
(5) temperature stability: essentially no impairment of the enzymes' activity by incubation of the enzymes at temperatures up to 40°C for 30 minutes; the activity of the enzymes remaining after incubation at 40°C for 30 minutes is at least 90%.

6. Process for the preparation of a DNA sequence coding for an alkaline Bacillus lipase with an aminoacid sequence which displays at least 70% homology with the amino-acid sequence indicated in Fig. 1, by cultivation of a Bacillus strain which produces the lipase with the indicated aminoacid sequence, and isolation of the DNA sequence coding for the lipase.

7. Process for the preparation of a DNA sequence according to Claim 6, characterised in that a DNA sequence which codes for an aminoacid sequence which displays at leat 80%, preferably at least 90%, homology with the aminoacid sequence indicated in Fig. 1 is prepared.

8. Process for the preparation of a vector suitable for the transformation of microorganisms, preferably of an expression vector, containing a DNA sequence according to Claim 6 or 7, by introducing the DNA sequence coding for the lipase into a vector DNA.

9. Process for the preparation of transformed microorganisms suitable for the expression of Bacillus lipases, characterised in that a vector according to Claim 8 is transformed into a suitable microorganism.

10. Process for the preparation of a transformed microorganism according to Claim 9, characterised in that a Bacillus subtilis, Bacillus alcalophilus, Bacillus licheniformis or Bacillus amyloliquefaciens is transformed.

11. Process for the preparation of a Bacillus pumilus of the species DSM 5776, DSM 5777 and DSM 5778 by cultivation of the natural isolates of Bacillus deposited at the Deutsche Sammlung von Mikroorganismen, Federal Republic of Germany, with the numbers DSM 5776, DSM 5777 or DSM 5778.

12. Compositions for washing, cleaning, bleaching or dishwashing, containing an alkaline Bacillus lipase which is obtainable according to a process of Claims 1 to 5.

13. Compositions according to Claim 12, characterised in that they contain the alkaline Bacillus lipase in the presence of protease.

14. Compositions according to Claim 12 or 13, characterised in that they contain the alkaline Bacillus lipase in a formulation for low use temperatures, preferably for use temperatures from about 30 to 40°C.

## Revendications (Revendications pour l'(les) Etat(s) contractant(s) suivant(s): AT, BE, CH, DE, DK, FR, GB, GR, IT, LI, LU, NL, SE)

1. Lipases de bacillus ayant un optimum de pH sur l'intervalle des pH alcalins et un optimum de température dans l'intervalle d'environ 30 à 40°C, que l'on peut obtenir par culture de Bacillus pumilus DSM 5776, DSM 5777 ou DSM 5778.

2. Lipases bacillaires alcalines selon la revendication 1, caractérisées en ce qu'elles comportent une séquence d'acides aminés présentant une homologie à au moins 70 % avec la séquence d'acides aminés ressortant de la Figure 1.

3. Lipases bacillaires alcalines selon la revendication 2, caractérisées en ce que leur séquence d'acides aminés comporte une homologie d'au moins 80 % avec la séquence d'acides aminés présentée sur la Figure 1.

4. Lipases bacillaires alcalines selon l'une des revendications 1 à 3, présentant les propriétés suivantes :
(1) Action : dégradation des triacylglycérides et des esters d'acides gras ;
(2) Optimum de pH : à pH environ 9 à 10 ;
(3) Stabilité au pH : à pH 6,5 à 11, les enzymes se révèlent parfaitement stables ; après incubation à pH 11 et 4°C pendant 21 heures, l'activité résiduelle des enzymes est d'au moins 90 % ;
(4) Optimum de température : environ 30 à 40°C ;
(5) Stabilité à la température : une incubation des enzymes à des températures allant jusqu'à 40°C pendant 30 minutes n'influe pas d'un manière négative sur l'activité des enzymes ; après 30 minutes d'incubation à 40°C, l'activité résiduelle des enzymes est d'au moins 90 %.

5. Séquence d'ADN codant pour une lipase bacillaire alcaline ayant une séquence d'acides aminés ayant une homologie d'au moins 70 % avec la séquence d'acides aminés présentée sur la Figure 1.

6. Séquence d'ADN selon la revendication 5, caractérisée en ce qu'elle code pour une séquence d'acides aminés ayant une homologie d'au moins 80 % et de préférence d'au moins 90 % avec la séquence d'acides aminés présentée sur la Figure 1.

7. Vecteur convenant à la transformation de microorganismes, de préférence vecteur d'expression, contenant une séquence d'ADN selon les revendications 5 ou 6.

8. Microorganismes transformés, convenant à l'expression de lipases bacillaires, caractérisés en ce qu'ils sont transformés à l'aide d'un vecteur selon la revendication 7.

9. Microorganismes transformés selon la revendication 8, caractérisés en ce qu'il s'agit de l'un des microorganismes transformés Bacillus subtilis, Bacillus alcalophilus, Bacillus licheniformis ou Bacillus amyloliquefaciens.

10. Bacillus pumilus, des espèces DSM 5776, DSM 5777 et DSM 5778.

11. Compositions pour laver, nettoyer, blanchir ou laver la vaisselle, contenant une lipase bacillaire alcaline selon l'une des revendications 1 à 4.

12. Compositions selon la revendication 11, caractérisées en ce qu'elles contiennent la lipase bacillaire alcaline en présence d'une protéase.

13. Compositions selon les revendications Il ou 12, caractérisées en ce qu'elles contiennent la lipase bacillaire alcaline dans une formulation destinée à de basses températures d'utilisation, de préférence à des températures d'utilisation d'environ 30 à 40°C.

14. Procédé de préparation des lipases bacillaires alcalines selon l'une des revendications 1 à 4, caractérisé en ce qu'on cultive un microorganisme selon l'une des revendications 8 et 9 ou un Bacillus pumilus, puis qu'on isole la lipase bacillaire alcaline formée.

15. Procédé selon la revendication 14, caractérisé en ce qu'on cultive un Bacillus pumilus des espèces DSM 5576, DSM 5777 ou DSM 5778.

## Revendications (Revendications pour l'(les) Etat(s) contractant(s) suivant(s): ES)

1. Procédé de préparation des lipases ayant un optimum de pH sur l'intervalle de pH alcalin et un optimum de température sur l'intervalle d'environ 30 à 40°C, caractérisé en ce qu'on cultive un Bacillus pumilus ou un microorganisme transformé par un vecteur d'expression - contenant une séquence d'ADN codant pour une lipase bacillaire alcaline ayant une séquence d'acides aminés ayant une homologie d'au moins 70 % avec la séquence d'acides aminés indiquée sur la Figure 1 - de préférence un microorganisme transformé Bacillus subtilis, Bacillus alcalophilus, Bacillus licheniformis ou Bacillus amyloliquefaciens, puis qu'on isole la lipase bacillaire alcaline formée.

2. Procédé selon la revendication 1, caractérisé en ce qu'on cultive un Bacillus pumilus des espèces DSM 5576, DSM 5777 ou DSM 5778.

3. Procédé de préparation des lipases bacillaires alcalines selon la revendication 2, caractérisé en ce qu'on prépare des lipases ayant une séquence d'acides aminés présentant une homologie d'au moins 70 % avec la séquence d'acides aminés présentée sur la Figure 1.

4. Procédé de préparation des lipases bacillaires alcalines selon la revendication 3, caractérisé en ce qu'on prépare des lipases dont la séquence d'acides aminés a une homologie d'au moins 80 % avec la séquence d'acides aminés présentée sur la Figure 1.

5. Procédé de préparation des lipases bacillaires alcalines selon l'une des revendications 1 à 4, caractérisé en ce qu'on prépare des lipases ayant les propriétés suivantes :
(1) Action : dégradation des triacylglycérides et des esters d'acides gras ;
(2) Optimum de pH : à pH environ 9 à 10 ;
(3) Stabilité au pH : à pH 6,5 à 11, les enzymes se révèlent parfaitement stables ; après incubation à pH Il à 4°C pendant 21 heures, l'activité résiduelle des enzymes est d'au moins 90 % ;
(4) Optimum de température : environ 30 à 40°C;
(5) Stabilité à la température : une incubation des enzymes à des températures allant jusqu'à 40°C pendant 30 minutes n'influe pas d'un manière négative sur l'activité des enzymes ; après 30 minutes d'incubation à 40°C, l'activité résiduelle des enzymes est d'au moins 90 %.

6. Procédé de préparation d'une séquence d'ADN codant pour une lipase bacillaire alcaline ayant une séquence d'acides aminés présentant une homologie d'au moins 70 % avec la séquence d'acides aminés ressortant de la Figure 1, par culture d'une souche bacillaire qui produit la lipase ayant la séquence d'acides aminés indiquée, et isolement de la séquence d'ADN codant pour la lipase.

7. Procédé de préparation d'une séquence d'ADN selon la revendication 6, caractérisé en ce qu'on prépare une séquence d'ADN codant pour une séquence d'acides aminés présentant une homologie d'au moins 80 % et de préférence d'au moins 90 % avec la séquence d'acides aminés ressortant de la Figure 1.

8. Procédé de préparation d'un vecteur convenant à la transformation de microorganismes, de préférence un vecteur d'expression, contenant une séquence d'ADN selon les revendications 6 ou 7, par incorporation de la séquence d'ADN codant pour la lipase dans un ADN vecteur.

9. Procédé de préparation de microorganismes transformés, convenant à l'expression de lipases bacillaires, caractérisé en ce qu'on transforme un vecteur selon la revendication 8 dans un microorganisme approprié.

10. Procédé de préparation d'un microorganisme transformé selon la revendication 9, caractérisé en ce qu'on transforme un Bacillus subtilis, Bacillus alcalophilus, Bacillus licheniformis ou Bacillus amyloliquefaciens.

11. Procédé de préparation d'un Bacillus pumilus de l'espèce DSM 5776, DSM 5777 et DSM 5778 par culture des isolats naturels de Bacillus déposés auprès de la Deutsche Sammlung von Mikroorganismem, République Fédérale d'Allemagne, sous les numéros DSM 5776, DSM 5777 ou DSM 5778.

12. Compositions pour laver, nettoyer, blanchir ou laver la vaisselle, contenant une lipase bacillaire alcaline selon l'une des revendications 1 à 5.

13. Compositions selon la revendication 12, caractérisées en ce qu'elles contiennent la lipase bacillaire alcaline en présence d'une protéase.

14. Compositions selon les revendications 12 ou 13, caractérisées en ce qu'elles contiennent la lipase bacillaire alcaline dans une formulation destinée à de basses températures d'utilisation, de préférence à des températures d'utilisation d'environ 30 à 40°C.
